(19) Europäisches Patentamt
European Patent Office
Office européen des brevets

(11) EP 4 211 184 B1

(12) EUROPÄISCHE PATENTSCHRIFT

(45) Veröffentlichungstag und Bekanntmachung des Hinweises auf die Patenterteilung:
**13.03.2024 Patentblatt 2024/11**

(21) Anmeldenummer: **20771274.6**

(22) Anmeldetag: **09.09.2020**

(51) Internationale Patentklassifikation (IPC):
***C08G 77/26*** (2006.01) ***C08G 77/388*** (2006.01)
***C08L 83/08*** (2006.01) ***C09D 183/08*** (2006.01)
***D06M 15/643*** (2006.01)

(52) Gemeinsame Patentklassifikation (CPC):
**C08G 77/388; C09D 183/08; D06M 15/643; D06M 15/6436; D06M 15/647; D06M 15/657;**
C08G 77/26; D06M 2200/50

(86) Internationale Anmeldenummer:
**PCT/EP2020/075247**

(87) Internationale Veröffentlichungsnummer:
**WO 2022/053137 (17.03.2022 Gazette 2022/11)**

(54) **VERWENDUNG VON CYCLENARMEN DERIVATISIERTEN AMINOFUNKTIONELLEN SILICONPOLYMEREN ZUR BEHANDLUNG VON FASERIGEN SUBSTRATEN**

USE OF LOW-CYCLEN DERIVATIZED AMINO-FUNCTIONAL SILICONE POLYMERS FOR TREATING FIBROUS SUBSTRATES

UTILISATION DE POLYMÈRES DE SILICONE À FONCTION AMINO DÉRIVÉS À FAIBLE CYCLÈNE POUR LE TRAITEMENT DE SUBSTRATS FIBREUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**19.07.2023 Patentblatt 2023/29**

(73) Patentinhaber: **Wacker Chemie AG**
**81737 München (DE)**

(72) Erfinder:
- **HELLER, Anton**
  **84359 Simbach (DE)**
- **GRÜNER, Elisabeth**
  **84533 Haiming (DE)**

(56) Entgegenhaltungen:
**EP-A1- 0 856 553**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 211 184 B1

## Beschreibung

Die Erfindung betrifft die Verwendung von Zusammensetzungen zur hydrophilen und im Weichgriff waschbeständigen Ausrüstung von faserigen Substraten, enthaltend cyclenarme Derivate von aminofunktionellen Organopolysiloxanen.

Marktgängige siliconhaltige Weichgriffmittel zur hydrophilen Ausrüstung von faserigen Materialien enthalten in den meisten Fällen amino- oder ammoniumfunktionalisierte Siliconpolyether, die mehrstufig und über den teuren Syntheseweg der Hydrosilylierung hergestellt werden müssen. In der Patentliteratur finden sich Beispiele, in denen Si-H funktionelle Polydiorganosiloxane mit epoxyfunktionellen olefinischen Verbindungen unter Katalyse mit platinhaltigen Verbindungen umgesetzt werden. In einer zweiten Reaktionsstufe folgt die Epoxy-Ringöffnung mit aminofunktionellen Polyetherverbindungen. Exemplarisch hierfür können die Schriften US 5981681 A, US 7041767 B2 oder US 7897716 B2 angeführt werden. Ein anderer Weg zu amino- oder ammoniumfunktionalisierten Siliconpolyethern führt über die Hydrosilylierung von Allylpolyethern und anschließender Anbindung von Amin oder Ammonium über Diisocyanate an die Hydroxylendgruppe des Polyethers. Dies wird in US 2008/0075683 A1 beschrieben. Neben dem Nachteil der teuren Herstellung sind die hydrophilen polyetherhaltigen Produkte nicht waschresistent, und sie zeigen teilweise eine störende Eigenfarbe wegen des Platinkatalysators.

US 6576606 B2 beschreibt eine kostengünstige Möglichkeit für den Zugang zu siliconhaltigen Weichgriffmittel zur hydrophilen Ausrüstung von faserigen Materialien ohne silicongebundenen Polyether. End- und seitenständig aminofunktionalisiertes Polydiorganosiloxan wird mit Essigsäureanhydrid weitestgehend vollständig acetyliert. Damit ausgerüstete textile Materialien zeigen gute Saugfähigkeit und geringe Vergilbung, jedoch einen nur moderaten Weichgriff und keine Waschpermanenz.

Eine teilweise Acetylierung von aminofunktionellen Polydiorganosiloxanen verbessert zwar den Weichgriff und die Waschfestigkeit, führt aber zu einer nicht akzeptablen Reduzierung der hydrophilen Eigenschaften textiler Materialien.

Nach der Einstufung von Cyclen D4, D5 und D6 als substances of very high concern (SVHC) durch die EU-Kommission gewinnt die Forderung, dass Verunreinigungen durch cyclische Oligodimethylsiloxan-Anteile mit 4 bis 6 Siloxaneinheiten (Cyclen D4, D5 und D6) in Polydiorganosiloxan-Polymeren nur unter einer Konzentration von jeweils 1000 ppm enthalten sein dürfen, zunehmende Bedeutung im Markt. Dies schließt auch ein, dass sich derartige cyclische Oligodimethylsiloxane innerhalb einer Haltbarkeitsperiode, in der Regel zwischen 0,5 und 2 Jahren, nicht derart nachbilden, dass diese geforderte Schwelle überschritten wird.

Bei Acetylierungen von aminofunktionellen Polydiorganosiloxanen mit Essigsäureanhydrid wird freie Essigsäure als Nebenprodukt gebildet, deren Entfernung aus anwendungstechnischer Sicht nicht notwendig ist. In der Regel werden wässrige Formulierungen von aminofunktionellen Polydiorganosiloxanen ohnehin mit Essigsäure auf pH-Werte kleiner gleich 7 eingestellt. Die Erfahrung zeigt jedoch, dass bei Anwesenheit von freier Essigsäure in aminofunktionellen Polydimethylsiloxanen die genannten cyclischen Oligodimethylsiloxane innerhalb der Haltbarkeiten in Mengen von weit über die Schwelle von 1000 ppm hinaus nachgebildet werden.

Neben der Acetylierung mit Essigsäureanhydrid gibt es auch andere Möglichkeiten der Acylierung. US 4978561 A beschreibt Acylierungen von aminofunktionellen Siliconen mit Lactonen. In der Anwendung auf fasrigen Materialien führen diese amidofunktionellen Silicone zu weniger Vergilbung des Substrats als ihre aminofunktionellen Vorstufen. Hydrophile Eigenschaften werden nicht beschrieben. In den Beispielen werden nur trimethylsilyl-terminierte aminofunktionelle Polydimethylsiloxane mit Lactonen umgesetzt, während hydroxy- oder alkoxyendständige Polymere über die Kondensation von amidofunktionellen Silanen erzeugt werden.

Umsetzungen von Aminosiliconen mit Lactonen sowie Alkylencarbonaten werden in US 5174813 A für den Einsatz in Poliermitteln oder in US 5389364 A zum Einsatz in Haarspülungsformulierungen beschrieben.

US 5824814 A beansprucht eine Methode zur Herstellung von viskositätsstabilen amidofunktionellen Polysiloxanen, bei der die aminofunktionellen Vorstufen unter Verwendung von Alkoholen oder substituierten Alkoholen vor der weiteren Umsetzung mit Lactonen abgestoppert werden. Cyclenarme Produkte lassen sich über diesen Weg nicht herstellen, ohne dass Alkohol beim Aufreinigen am Dünnschichtverdampfer abgespalten und entfernt wird.

KR 20070072069 A beschreibt die Umsetzung von aminofunktionellen Polysiloxanen mit Glycerincarbonat, wobei die Viskositätsstabilität durch Zusatz von Laurylethoxylaten erreicht werden soll. In den Beispielen sollen bereits trimethylsilyl-terminierte aminofunktionelle Polysiloxane mit Glycerincarbonat und Laurylethoxylat bei 30°C bis 80°C ohne Zusatz eines Äquilibrierungskatalysators über 1 bis 12 Stunden umgesetzt werden. Der Erfolg der Umsetzung ist aber nicht durch Analysedaten belegt. In der Anwendung auf textilen Fasersubstraten werden im Vergleich zur aminofunktionellen Vorstufe, sowie dem entsprechenden Umsetzungsprodukt dieser Vorstufe mit $\gamma$-Butyrolacton verbesserte Dochteffekte beim Eintauchen in Wasser gesehen.

Das Dokument EP 0 856 553 A1 liegt im selben technischen Gebiet als die Anmeldung und offenbart die Verwendung von Monohydroxyamido- oder Monohydroxycarbamatopolysiloxanen, die auch hydrolisierbare, bzw. Hydroxygruppen aufweisen.

Die Aufgabe, die zu dieser Erfindung geführt hat, bestand in der Bereitstellung von möglichst farblosen, kostengünstig herstellbaren Organopolysiloxan-haltigen Zusammensetzungen zur Ausrüstung von faserigen Substraten, z.B. textilen Fasermaterialien, die diesen Substraten hervorragenden Weichgriff, hydrophile Eigenschaften und gute Waschbeständigkeit bezüglich des Weichgriffs vermitteln. Für eine einfache Verarbeitbarkeit sollen die Komponenten der Organopolysiloxan-haltigen Zusammensetzungen vorzugsweise Viskositäten von kleiner als 5000 mPa·s bei 25°C aufweisen. Außerdem sollen diese Zusammensetzungen Oligodimethylsiloxan-Anteile mit 4 bis 6 Siloxaneinheiten (Cyclen D4 bis D6) unter einer Konzentration von jeweils 1000 ppm, bezogen auf das Organopolysiloxan-Polymer, enthalten und solche Cyclen bei Raumtemperatur-Lagerung so wenig wie möglich nachbilden. Diese Eigenschaft soll im Folgenden unter der Bezeichnung "cyclenarm" verstanden werden. Ihre Bedeutung ist den anderen Eigenschaften gleichzusetzen, da in vielen Fällen nicht cyclenarme Produkte nach Einführung der SVHC-Einstufung (substances of very high concern) aus Gesundheits- und Umweltschutzgründen nicht mehr eingesetzt werden.

Die Aufgabe wird durch die Erfindung gelöst. Gegenstand der Erfindung ist die Verwendung von Zusammensetzungen zur hydrophilen und im Weichgriff waschbeständigen Ausrüstung von faserigen Substraten enthaltend cylenarme Derivate von aminofunktionellen Organopolysiloxanen, enthaltend Siloxaneinheiten der allgemeinen Formel(I)

$$R_aZ_bSiO_{\frac{4-(a+b)}{2}} \quad (I),$$

wobei

a 0, 1 oder 2, vorzugsweise 0 oder 1, ist
b 1, 2 oder 3, vorzugsweise 1, ist
mit der Maßgabe, dass die Summe aus a+b ≤ 3 ist,
gegebenenfalls Siloxaneinheiten der allgemeinen Formel (II)

$$R_cQ_dSiO_{\frac{4-(c+d)}{2}} \quad (II),$$

wobei
c 0, 1 oder 2, vorzugsweise 0 oder 1, ist
d 1, 2 oder 3, vorzugsweise 1, ist
mit der Maßgabe, dass die Summe aus c+d ≤ 3 ist,
Siloxaneinheiten der allgemeinen Formel (III)

$$R_eSiO_{\frac{4-e}{2}} \quad (III),$$

wobei
e 0, 1, 2, oder 3, vorzugsweise 2, ist,
und Siloxaneinheiten der allgemeinen Formel (IV)

$$Y_fR_gSiO_{\frac{4-(f+g)}{2}} \quad (IV),$$

wobei
f 1, 2 oder 3, vorzugsweise 1, ist,
g 0, 1 oder 2, vorzugsweise 2, ist,
mit der Maßgabe, dass die Summe aus f+g ≤ 3 ist,
wobei R gleich oder verschieden sein kann, und ein Wasserstoffatom oder einen einwertigen, gegebenenfalls Fluor-, Chlor- oder Brom-substituierten $C_1$- bis $C_{18}$-Kohlenwasserstoffrest bedeutet,
Z eine Gruppe der allgemeinen Formel (V) bedeutet

$$-R^2-[NR^3(CH_2)_n]_iNR^4R^5 \quad (V),$$

wobei

i 0, 1, 2, 3 oder 4 ist,
n 2, 3, 4, 5 oder 6 ist,
$R^2$ einen zweiwertigen, linearen oder verzweigten $C_1$- bis $C_{18}$-Kohlenwasserstoffrest bedeutet,
$R^3$ ein Wasserstoffatom, einen gegebenenfalls Fluor-, Chlor-, Brom-, Hydroxy- oder $C_1$- bis $C_5$-alkoxysubstituierten $C_1$- bis $C_{18}$-Kohlenwasserstoffrest, einen Acylrest oder einen Rest der allgemeinen Formel (VI) bedeutet,
$R^4$ einen Rest der allgemeinen Formel (VI) bedeutet,

$$-(C{=}O)-R^6-OH \qquad (VI),$$

$R^5$ ein Wasserstoffatom oder einen gegebenenfalls Fluor-, Chlor-, Brom-, Hydroxy- oder $C_1$- bis $C_5$-alkoxysubstituierten $C_1$-bis $C_{18}$-Kohlenwasserstoffrest oder einen Acylrest bedeutet, $R^6$ einen zweiwertigen, linearen oder verzweigten $C_2$- bis $C_8$-Kohlenwasserstoffrest oder einen Rest $-OR^7-$ bedeutet,
$R^7$ einen zweiwertigen linearen oder verzweigten $C_2$ bis $C_8$-Kohlenwasserstoffrest bedeutet,
Q eine Gruppe der allgemeinen Formel (VII) bedeutet

$$-R^2-[NR^9(CH_2)_n]_iN(R^9)_2 \qquad (VII),$$

wobei
$R^2$, i und n die oben dafür angegebene Bedeutung haben,
$R^9$ gleich oder verschieden sein kann, und ein Wasserstoffatom, einen gegebenenfalls Fluor-, Chlor-, Brom-, Hydroxy- oder $C_1$-bis $C_5$-alkoxysubstituierten $C_1$- bis $C_{18}$-Kohlenwasserstoffrest oder einen Acylrest bedeutet,
Y einen Rest der allgemeinen Formel (VIII) und/oder (IX) bedeutet,

$$-OR^1 \text{ (VIII) und/oder } -[O(CHR^{10})_p]_mOR^{11} \qquad (IX),$$

wobei
m 0 oder eine ganze Zahl von 1 bis 100, vorzugsweise 1 bis 20, ist,
p 2, 3 oder 4 ist,
$R^1$ ein Wasserstoffatom oder einen $C_1$- bis $C_4$-Alkylrest bedeutet,
$R^{10}$ ein Wasserstoffatom oder einen $C_1$- bis $C_{18}$-Kohlenwasserstoffrest bedeutet,
$R^{11}$ ein Wasserstoffatom, einen $C_1$- bis $C_{10}$-Kohlenwasserstoffrest, vorzugsweise einen $C_4$- bis $C_{10}$-Kohlenwasserstoffrest, oder eine Gruppe der allgemeinen Formel $-(C{=}O)-R^{12}$ bedeutet,
$R^{12}$ einen $C_1$- bis $C_{10}$-Kohlenwasserstoffrest oder $O-R^{13}$ bedeutet und $R^{13}$ einen $C_1$- bis $C_{10}$-Kohlenwasserstoffrest bedeutet,
mit der Maßgabe, dass mindestens ein Teil der Reste Y Reste der Formel (IX) sind,
mit der Maßgabe, dass Verunreinigungen an
Octamethylcyclotetrasiloxan (Cyclen D4),
Decamethylcyclopentasiloxan (Cyclen D5) und Dodecamethylcyclohexasiloxan (Cyclen D6) in Anteilen von jeweils weniger als 0,1 Gew.-% enthalten sind und nach einer Lagerzeit von 20 Tagen bei einer Temperatur von 50°C die Anteile Cyclen D4, repräsentativ für D4, D5 und D6, immer noch kleiner sind als jeweils 0,1 Gew.-%, jeweils bezogen auf das Gesamtgewicht der Derivate von aminofunktionellen Organopolysiloxanen.

Der Begriff Organopolysiloxane soll im Rahmen der vorliegenden Erfindung sowohl polymere als auch dimere und oligomere Siloxane umfassen.

Beispiele für Kohlenwasserstoffreste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste, und Aralkylreste, wie der Benzylrest, der $\alpha$- und der $\beta$-Phenylethylrest.

Die Kohlenwasserstoffreste R enthalten gegebenenfalls eine aliphatische Doppelbindung. Beispiele sind Alkenylreste, wie der Vinyl-, Allyl-, 5-Hexen-1-yl-, E-4-Hexen-1-yl-, Z-4-Hexen-1-yl-, 2-(3-Cyclohexenyl)-ethyl- und Cyclododeca-4,8-dienyl-rest. Bevorzugte Reste R mit aliphatischer Doppelbindung sind der Vinyl-, Allyl-, und 5-Hexen-1-ylrest. Vorzugsweise enthalten jedoch höchstens 1% der Kohlenwasserstoffreste R eine Doppelbindung.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Bevorzugt handelt es sich bei dem Rest R um einen einwertigen Kohlenwasserstoffrest mit 1 bis 6 Kohlenstoffatomen, wobei der Methylrest besonders bevorzugt ist.

Beispiele für Kohlenwasserstoffreste $R^1$ sind Alkylreste, wie der Methyl-, Ethyl-, n-Propyl-, iso-Propyl-, 1-n-Butyl-, 2-n-Butyl-, iso-Butyl- und tert.-Butylrest, wobei $C_1$-$C_3$-Alkylreste bevorzugt sind.

Beispiele für durch einen Ethersauerstoff substituierte Alkylreste $R^1$ sind der Methoxyethyl- und der Ethoxyethylrest. Bevorzugte Beispiele für den Rest $-OR^1$ sind der Hydroxy- und Methoxyrest.

Vorzugsweise ist $R^2$ ein zweiwertiger $C_2$-$C_6$-Kohlenwasserstoffrest.

Beispiele für zweiwertige Kohlenwasserstoffreste $R^2$ sind gesättigte lineare, verzweigte und cyclische Alkylenreste, wie der Methylen- und Ethylenrest sowie Propylen-, Butylen-, Pentylen-, Hexylen-, 2-Methylpropylen-, Cyclohexylen- und Octadecylenreste, oder ungesättigte Alkylen- oder Arylenreste, wie der Hexenylenrest und Phenylenreste, wobei der n-Propylenrest und der 2-Methylpropylenrest besonders bevorzugt sind.

Beispiele für Kohlenwasserstoffreste R, ausgenommen solche mit aliphatischer Doppelbindung, gelten in vollem Umfang für Kohlenwasserstoffreste $R^3$, $R^5$ und $R^9$. Vorzugsweise sind $R^3$, $R^5$, und $R^9$ ein Wasserstoffatom, ein Methylrest, ein Cyclohexylrest, oder ein Acylrest, wie der Acetylrest.

Bevorzugte Alkylenreste für $R^6$ sind der Ethylen-, Propylen-, Butylen- und Pentylenrest; besonders bevorzugt ist der Propylenrest.

Bevorzugte Alkylenreste für $R^7$ sind der Ethylen-, Isopropylen-und der Ethylethylenrest; besonders bevorzugt ist der Isopropylenrest.

Bevorzugte Beispiele für Q sind
$H_2N(CH_2)_3$-
$H_2N(CH_2)_2NH(CH_2)_3$-
$H_2N(CH_2)_2NH(CH_2)CH(CH_3)CH_2$-
(Cyclohexyl)$NH(CH_2)_3$-
$CH_3NH(CH_2)_3$-
$(CH_3)_2N(CH_2)_3$-
$CH_3CH_2NH(CH_2)_3$-
$(CH_3CH_2)_2N(CH_2)_3$-
$CH_3NH(CH_2)_2NH(CH_2)_3$-
$(CH_3)_2N(CH_2)NH(CH_2)_3$-
$CH_3CH_2NH(CH_2)_2NH(CH_2)_3$-
$(CH_3CH_2)_2N(CH_2)_2NH(CH_2)_3$-
und deren teil- oder vollacetylierte Formen.

Besonders bevorzugte Beispiele für Q sind:

$H_2N(CH_2)_3$-
$H_2N(CH_2)_2NH(CH_2)_3$- und
(Cyclohexyl)$NH(CH_2)_3$-

Bevorzugt werden in den erfindungsgemäßen Zusammensetzungen cyclenarme Derivate von aminofunktionellen Organopolysiloxanen der allgemeinen Formel (X) eingesetzt.

$$YR_2SiO\text{-}(R_2SiO)_k\text{-}(RZSiO)_l\text{-}(RQSiO)_o\text{-}R_2SiY \qquad (X),$$

wobei

R, Y, Z und Q die oben dafür angegebenen Bedeutungen haben,
k eine ganze Zahl von 50 bis 700,
1 eine ganze Zahl von 1 bis 30, vorzugsweise 1 bis 10,
besonders bevorzugt 1 bis 5 und
o eine ganze Zahl von 0 bis 30, bevorzugt 1 bis 15, besonders bevorzugt 1 bis 6, bedeuten,
mit der Maßgabe, dass mindestens ein Teil der Reste Y Reste der Formel (IX) sind.

Vorzugsweise sind mindestens 1 Mol%, bevorzugt mindestens 5 Mol%, und höchstens 99 Mol%, bevorzugt höchstens 60 Mol%, der Reste Y Reste der Formel (IX).

Im Rahmen dieser Erfindung soll Formel (X) so verstanden werden, dass k Einheiten $-(R_2SiO)-$, 1 Einheiten -(RZSiO)- und o Einheiten -(RQSiO)- in beliebiger Weise, beispielsweise als Block oder statistisch, im Organopolysiloxanmolekül verteilt sein können.

Entspricht der Rest Y in den erfindungsgemäßen Derivaten von aminofunktionellen Organopolysiloxanen, vorzugsweise in denen der Formel (X), einem Rest der Formel (VIII), so handelt es sich vorzugsweise um einen Hydroxyl- und/oder Methoxyrest. Ein Teil der Reste Y in den erfindungsgemäßen Derivaten von aminofunktionellen Organopolysiloxanen, vorzugsweise in denen der Formel (X), entspricht (Iso)Oxyalkylresten der Formel (IX), wobei es sich vorzugsweise um $C_4$-$C_{10}$-Alkoxyreste oder $C_4$-$C_{10}$-Monoalkylglykoletherreste, bevorzugt um $C_4$-$C_{10}$-Monoalkylglykol-etherreste, handelt, neben Resten der Formel (VIII), die vorzugsweise Hydroxyl- und/oder Methoxyreste sind.

Innerhalb bzw. entlang der Siloxankette der Siloxane der oben angegebenen Formel können, was durch derartige Formeln üblicherweise nicht dargestellt wird, zusätzlich zu den Diorganosiloxaneinheiten $R_2SiO$ noch andere Siloxaneinheiten vorliegen. Beispiele für solche anderen, meist nur als Verunreinigung vorliegende Siloxaneinheiten sind solche der Formeln $RSiO_{3/2}$, $R_3SiO_{1/2}$ und $SiO_2$, wobei R die oben dafür angegebene Bedeutung hat.

Ein weiterer Gegenstand der Erfindung ist ein Verfahren zur Herstellung der in den erfindungsgemäßen Zusammensetzungen eingesetzten cyclenarmen Derivate von aminofunktionellen Organopolysiloxanen, indem aminofunktionelle Organopolysiloxane (1) enthaltend aminofunktionelle Siloxaneinheiten der allgemeinen Formel (II)

$$R_cQ_dSiO_{\frac{4-(c+d)}{2}} \qquad (II),$$

wobei Q, R, c und d die oben dafür angegebene Bedeutung haben,
und Siloxaneinheiten der allgemeinen Formel (XII)

$$R_g(OR^1)_fSiO_{\frac{4-(f+g)}{2}} \qquad (XII),$$

wobei R, $R^1$, f und g die oben dafür angegebene Bedeutung haben,
mit Verbindungen (2) der allgemeinen Formel (XI)

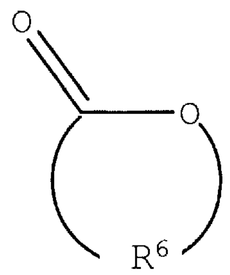

(XI),

wobei
$R^6$ die oben dafür angegebenen Bedeutungen hat,
und Verbindungen (3) der allgemeinen Formel (XIII)

$$H\text{-}[O(CHR^{10})_p]_mOR^{11} \qquad (XIII),$$

wobei
$R^{10}$, $R^{11}$, m und p die oben dafür angegebene Bedeutung haben, umgesetzt werden,
mit der Maßgabe, dass in den erhaltenen Derivaten von aminofunktionellen Organopolysiloxanen der Gehalt an Cyclen D4, D5 und D6 jeweils kleiner als 0,1 Gew.-% ist, indem die Cyclen vor, während oder nach der Umsetzung destillativ entfernt werden.

Beispiele für Verbindungen (2) der allgemeinen Formel (XI) sind Lactone oder cyclische Kohlensäureester.

Beispiele für Lactone sind β- und γ-Butyrolacton, γ-Valerolacton, α-Angelicalacton, δ-Valerolacton und ε-Caprolacton. Ein bevorzugtes Beispiel für ein Lacton ist γ-Butyrolacton. Beispiele für cyclische Kohlensäureester sind Ethylencarbonat, Propylencarbonat, 1,2-Butylencarbonat und 1,2-Hexylencarbonat. Ein bevorzugtes Beispiel für einen cyclischen Kohlensäureester ist Propylencarbonat.

Die Lactone oder cyclischen Kohlensäureester werden in Mengen von 3 bis 100 Mol%, vorzugsweise 6 bis 90 Mol%, besonders bevorzugt 14 bis 83 Mol%, jeweils bezogen auf den derivatisierbaren Amingehalt der eingesetzten

aminofunktionellen Organopolysiloxane (1) zur Herstellung der cyclenarmen derivatisierten aminofunktionellen Organopolysiloxane, eingesetzt.

Überraschenderweise bewirkt die Verwendung dieser hydroxyfreien Lactone und cyclischen Kohlensäureester in den erfindungsgemäßen Zusammensetzungen, dass die faserigen Substrate nach Anwendung der erfindungsgemäßen Zusammensetzungen schneller benetzbar werden als nach Verwendung hydroxy-haltiger Lactone, wie z.B. Gluconolacton, oder entsprechender hydroxy-haltiger Kohlensäureester, wie z.B. Glycerincarbonat.

Beispiele für Verbindungen (3) der allgemeinen Formel (XIII) sind Alkohole und Monoalkylglykolether. Beispiele für Alkohole sind Methanol und unter Synthesebedingungen nichtflüchtige, bei Raumtemperatur flüssige $C_6$- bis $C_{10}$-Alkohole, wie n-Hexanol, n-Heptanol, n-Octanol, 2-Ethylhexanol, n-Decanol, und 2-Methoxypropanol und 2-Butoxyethanol.

Beispiele für Monoalkylglykolether sind Addukte von Alkohol, Ethylenoxid, Propylenoxid und deren Mischpolymerisaten.

Bevorzugte Beispiele für Monoalkylglykolether sind Diethylenglykolmonobutylether, Dipropylenglykolmonomethylether, n-Hexylglykol und Propylenglykol-monobutylether, wobei Diethylenglykolmonobutylether und Dipropylenglykolmonomethylether besonders bevorzugte Beispiele sind.

Die Alkohole oder Addukte von Alkoholen (3) werden in Mengen von 1 bis 50 Gewichtsprozent, bevorzugt von 1 bis 10 Gewichtsprozent, besonders bevorzugt von 2 bis 7 Gewichtsprozent, jeweils bezogen auf Gesamtgewicht der Komponenten zur Herstellung der cyclenarmen derivatisierten aminofunktionellen Organopolysiloxane, eingesetzt.

Der Zusatz von Alkoholen mit kleiner oder gleich 10 Kohlenstoffatomen oder deren Addukten bewirkt zum Einen, dass bei der Herstellung der cylenarmen derivatisierten aminofunktionellen Organopolysiloxane Viskositäten bei 25°C von vorzugsweise kleiner als 5000 mPa·s, bevorzugt 500 bis 5000 mPa·s erreicht werden, zum Anderen, dass im Falle einer Umsetzung aller 3 Komponenten gleichzeitig in einer Synthesestufe die Mischbarkeit des aminofunktionellen Organopolysiloxans mit den eingesetzten Lactonen oder cyclischen Kohlenstoffestern verbessert wird.

Die Umsetzungen erfolgen bei Temperaturen von 10°C bis 130°C, bevorzugt von 50°C und 120°C, besonders bevorzugt von 60°C bis 90°C. Werden bereits cyclenarme aminofunktionelle Organopolysiloxane eingesetzt, ist es unerheblich, ob die Reaktionen von aminofunktionellen Organopolysiloxanen (1) mit Verbindungen (2) der allgemeinen Formel (XI) und Verbindungen (3) der allgemeinen Formel (XIII) gleichzeitig durchgeführt werden, oder zuerst die Reaktion von aminofunktionellen Organopolysiloxanen (1) mit Verbindungen (2) der allgemeinen Formel (XI) und danach die Reaktion mit Verbindungen (3) der allgemeinen Formel (XIII) erfolgt oder die Reaktionen in umgekehrter Reihenfolge erfolgen. Wenn eine Entfernung von Cyclen D4, D5 und D6 aus den derivatisierten aminofunktionellen Siliconpolymeren, vorzugsweise am Dünnschichtverdampfer, erforderlich ist, sollten die Verfahrensschritte vorzugsweise in folgender Reihenfolge erfolgen: Zuerst erfolgt die Reaktion von aminofunktionellen Organopolysiloxanen (1) mit Verbindungen (2) der allgemeinen Formel (XI), dann erfolgt die destillative Entfernung der Cyclen D4, D5 und D6, vorzugsweise die Aufreinigung mittels Kurzwegdestillation oder Dünnschichtverdampfer, und zum Schluss wird, vorzugsweise im Abkühlprozess, die Umsetzung mit Verbindungen (3) der allgemeinen Formel (XIII) durchgeführt, was den Vorteil hat, dass damit kein Verlust von Verbindungen (3) der allgemeinen Formel (XIII) einhergeht.

Bevorzugt werden in dem Verfahren zur Herstellung von cylenarmen Derivaten von aminofunktionellen Organopolysiloxanen als aminofunktionelle Organopolysiloxane (1) solche der allgemeinen Formel (XIV)

$$R^1OR_2SiO-(R_2SiO)_k-(RQSiO)_{l+o}-SiR_2OR^1 \qquad (XIV),$$

wobei
R, $R^1$, k, 1 und o die jeweils oben dafür angegebene Bedeutung haben, eingesetzt.

Die erfindungsgemäßen Zusammensetzungen zur hydrophilen und im Weichgriff waschbeständigen Ausrüstung von faserigen Substraten, enthaltend cyclenarme Derivate von aminofunktionellen Organopolysiloxanen, können Lösungen dieser Derivate von aminofunktionellen Organopolysiloxanen in organischen Lösungsmitteln sein.

Vorzugsweise sind die erfindungsgemäßen Zusammensetzungen zur hydrophilen und im Weichgriff waschbeständigen Ausrüstung von faserigen Substraten wässrige Emulsionen, enthaltend erfindungsgemäße cyclenarme Derivate von aminofunktionellen Organopolysiloxanen (A)

Emulgatoren (B) und/oder Coemulgatoren (B') und
Wasser (C).
Gegenstand der Erfindung sind daher wässrige Emulsionen zur hydrophilen und im Weichgriff waschbeständigen Ausrüstung von faserigen Substraten, enthaltend
erfindungsgemäße cyclenarme Derivate von aminofunktionellen Organopolysiloxanen (A)
Emulgatoren (B) und/oder Coemulgatoren (B') und
Wasser (C).

Als Emulgatoren (B) können nicht-ionische, anionische oder kationische Emulgatoren oder auch deren Gemische eingesetzt werden.

Die erfindungsgemäßen wässrigen Emulsionen zur hydrophilen und im Weichgriff waschbeständigen Ausrüstung von faserigen Substraten enthalten an sich bekannte Emulgatoren und deren Mischungen.

Als anionische Emulgatoren eignen sich besonders:

1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid(PO)einheiten.

2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.

3. Alkali- und Ammoniumsalze von Carbonsäuren mit 8 bis 20 C-Atomen im Alkyl-, Aryl-, Alkaryl- oder Aralkylrest.

4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Als nicht-ionische Emulgatoren eignen sich besonders:

5. Polyvinylalkohol, der noch 5 bis 50 %, vorzugsweise 8 bis 20 % Vinylacetateinheiten aufweist, mit einem Polymerisationsgrad von 500 bis 3000.

6. Alkylpolyglykolether, vorzugsweise solche mit 8 bis 40 EO-Einheiten und Alkylresten von 8 bis 20 C-Atomen.

7. Alkylarylpolyglykolether, vorzugsweise solche mit 8 bis 40 EO-Einheiten und 8 bis 20 C-Atomen in den Alkyl- und Arylresten.

8. Ethylenoxid/Propylenoxid(EO/PO)-Blockcopolymere, vorzugsweise solche mit 8 bis 40 EO- bzw. PO-Einheiten.

9. Additionsprodukte von Alkylaminen mit Alkylresten von 8 bis 22 C-Atomen mit Ethylenoxid oder Propylenoxid.

10. Fettsäuren mit 6 bis 24 C-Atomen.

11. Alkylpolyglykoside der allgemeinen Formel $R^*\text{-}O\text{-}Z_O$, worin $R^*$ einen linearen oder verzweigten, gesättigten oder ungesättigten Alkylrest mit im Mittel 8 - 24 C-Atomen und $Z_O$ einen Oligoglykosidrest mit im Mittel o = 1 - 10 Hexose- oder Pentoseeinheiten oder Gemischen davon bedeuten.

12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.

13. Polare Gruppen enthaltende lineare Organo(poly)siloxane, insbesondere solche mit Alkoxygruppen mit bis zu 24 C-Atomen und/oder bis zu 40 EO- und/oder PO-Gruppen.

Als kationische Emulgatoren eignen sich besonders:

14. Salze von primären, sekundären und tertiären Fettaminen mit 8 bis 24 C-Atomen mit Essigsäure, Schwefelsäure, Salzsäure und Phosphorsäuren.

15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.

16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als Ampholytische Emulgatoren eignen sich besonders:

17. Langkettig substituierte Aminosäuren, wie N-Alkyl-di- (aminoethyl-)glycin oder N-Alkyl-2-aminopropionsäuresalze.

18. Betaine, wie N-(3-Acylamidopropyl)-N,N-dimethylammonium-salze mit einem $C_8$-$C_{18}$-Acylrest und Alkyl-imidazolium-Betaine. Bevorzugt als Emulgatoren sind nichtionische Emulgatoren, insbesondere die vorstehend unter 6. aufgeführten Alkylpolyglykolether, die unter 9. aufgeführten Additionsprodukte von Alkylaminen mit Ethylenoxid oder Propylenoxid, die unter 11. aufgeführten Alkylpolyglykoside und die unter 5. aufgeführten Polyvinylalkohole.

Emulgatoren werden dabei in Mengen von 1 Gew.-% bis 70 Gew.-%, bezogen auf das Gesamtgewicht der wässrigen Emulsionen eingesetzt.

Die wässrigen Emulsionen zur hydrophilen und im Weichgriff waschbeständigen Ausrüstung von faserigen Substraten enthalten cylenarme Derivate von aminofunktionellen Organopolysiloxanen (A) vorzugsweise in Mengen von 0,5 Gew.-% bis 80 Gew.-%, bezogen auf das Gesamtgewicht der wässrigen Emulsionen.

Die erfindungsgemäßen wässrigen Emulsionen zur hydrophilen und im Weichgriff waschbeständigen Ausrüstung von faserigen Substraten können noch weitere Stoffe, wie Polyethylenglykole, Polypropylenglykole und Polyethylen-Polypropylenglykole und deren Mischungen, sowie Säuren, enthalten. Beispiele für Säuren sind Carbonsäuren, wie Essigsäure, Ameisensäure, Zitronensäure, Äpfelsäure und Milchsäure.

Die erfindungsgemäßen wässrigen Emulsionen zur hydrophilen und im Weichgriff waschbeständigen Ausrüstung von faserigen Substraten können als weitere Stoffe Lösungsmittel oder Co-Emulgatoren (B') enthalten.

Beispiele für nichtwässrige Lösungsmittel oder Co-Emulgatoren sind 1-Pentanol, 1-Hexanol, 1-Octanol, Propandiol, 1,3-Butandiol, 1,2-Hexandiol, 2-Ethylhexan-1,3-diol, 1,2-Octandiol, Glycerin, Diethylenglykolmethylether, Diethylenglykolethylether, Diethylenglykolmono-n-butylether, Propylenglykolmethylether.

Die bei der Herstellung der erfindungsgemäßen cylenarmen Derivate von aminofunktionellen Organopolysiloxanen zur hydrophilen und im Weichgriff waschbeständigen Ausrüstung von faserigen Substraten eingesetzten aminofunktionellen Organopolysiloxane (1) der Formel (XIV) können nach dem in US 7129369 B2 beschriebenen Verfahren hergestellt werden. Dem Fachmann sind auch andere Verfahren bekannt.

Zur Reduzierung der Cyclengehälter von D4, D5 und D6 bei den erfindungsgemäßen cylenarmen Derivaten von aminofunktionellen Organopolysiloxanen auf Konzentrationen von jeweils kleiner als 0,1 Gew.-% kann der Aufreinigungsprozess entweder bei den aminofunktionellen Organopolysiloxan-Vorstufen oder nach der 1. Umsetzungsstufe mit den hydroxyfreien Lactonen oder cyclischen Kohlensäureestern (2) erfolgen, bevor die Alkohole oder Alkoholaddukte (3) in der 2. Stufe eingesetzt werden. Bei dem Verfahren zur Herstellung der erfindungsgemäßen Zusammensetzung, enthaltend cyclenarme Derivate von aminofunktionellen Organopolysiloxanen, werden keine über den Messschwankungen erfassbaren Mengen von Cyclen D4, D5 oder D6 gebildet. Der Vorgang der Aufreinigung läuft in beiden Fällen vorzugsweise in gleicher Weise über einen handelsüblichen Dünnschichtverdampfer oder eine Kurzwegdestillationsanlage ab. Wahlweise kann zur schnelleren Vorreinigung eine Flash-Box vorgeschalten sein. Der Reinigungsprozess findet bei Temperaturen von vorzugsweise 100°C bis 200°C, bevorzugt von 140°C bis 190°C, besonders bevorzugt von 150°C bis 180°C, statt. Der Reinigungsprozess mittels Dünnschichtverdampfer findet vorzugsweise bei Drücken zwischen 0 und 200 mbar, bevorzugt zwischen 0 und 100 mbar, besonders bevorzugt zwischen 0 und 30 mbar, statt. Der Reinigungsprozess mittels Kurzwegdestillator findet vorzugsweise bei Drücken zwischen 0 und 20 mbar, bevorzugt zwischen 0 und 10 mbar, besonders bevorzugt zwischen 0 und 3 mbar, statt.

Beispiele für faserige Substrate, die mit den erfindungsgemäßen Zusammensetzungen, enthaltend cyclenarme Derivate von aminofunktionellen Organopolysiloxanen (A), behandelt werden, sind natürliche oder synthetisch hergestellte Fasern, Garne, Stränge, Seile, textile Flächengebilde wie Vliese, Matten, gewebte, geknüpfte, gewirkte oder gestrickte Textilien, Leder und Kunstleder, sowie Haare. Bevorzugte faserige Substrate sind Textilien. Für den Auftrag der erfindungsgemäßen Zusammensetzung können die Textilien in Form von Einzelfasern, Faserbündeln, Füllfasern, Garnen, Teppichen, Stoffbahnen oder Kleidungsstücken oder Teilen von Kleidungsstücken vorliegen.

Die Textilien können aus Baumwolle, Wolle, Mischpolymere von Vinylacetat, Rayon, Hanf, natürlicher Seide, Polypropylen, Polyethylen, Polyester, Polyurethan, Polyamid, Aramid, Polyimid, Polyacrylat, Polyacrylnitril, Polylactid, Polyvinylchlorid, Glasfasern, Keramikfasern, Cellulose oder deren Gemische bestehen.

Das Auftragen auf die zu behandelnden faserigen Substrate, bevorzugt Textilien, kann in beliebiger für die Behandlung von faserigen Substraten, wie Textilien, geeigneter und vielfach bekannter Weise, z.B. durch Tauchen, Streichen, Gießen, Sprühen, Aufwalzen, Klotzen, Drucken oder Schaumauftrag erfolgen.

In der Anwendung können die erfindungsgemäßen Zusammensetzungen mit gebrauchsüblichen Textilhilfsmitteln, wie Bindemitteln aus Melamin- oder Methylolharzen, Polyethylenen, Polyurethanen, Polyacrylaten, Polyvinylalkoholen, Polyvinylacetaten, optischen Aufhellern, Mattierungsmitteln, Elektrolyten, Benetzungshilfsmitteln, Plasten, Bleichmitteln, Antistatika, Dispersionen von Metalloxiden, Silikaten, Parfümölen, Farbstoffen, Konservierungsmitteln, Entschäumern oder weiteren Hydrophobier-und Oleophobierhilfsmitteln, z.B. perfluorierten Kohlenwasserstoffen, kombiniert werden.

Ferner können die erfindungsgemäßen Zusammensetzungen mit Textilweichmachern auf Basis von Polysiloxanen und organischen Weichmachern wie anionischen, kationischen und nichtionogenen Weichmachern und deren Mischungen verwendet werden.

Hierzu zählen funktionelle und nichtfunktionelle Silicone, Salze der Metallseifen, Alkylpolysulfonate, Sulfosuccinate und deren Derivate, Esterquats, Sulfoalkylenfettsäureamide, Alkylammoniumsulfate, Triethanolaminfettsäureester, Fettsäurepolyglycolester, Fettaminpolyalkylenaddukte, Fettsäureamidpolyalkylenaddukte, und Dispersionen von Paraffinen, Wachsen, Polyethylenen und Polyestern.

Die behandelten faserigen Substrate, bevorzugt Textilien, werden vorzugsweise bei Temperaturen von 20°C bis 200°C, bevorzugt 100°C bis 180°C, trocknen gelassen.

Die erfindungsgemäßen Zusammensetzungen, enthaltend cyclenarme Derivate von aminofunktionellen Organopolysiloxanen (A), haben den Vorteil, dass deren Verunreinigungen an Octamethylcyclotetrasiloxan (Cyclen D4), Decamethylcyclopentasiloxan (Cyclen D5) und Dodecamethylcyclohexasiloxan (Cyclen D6) in Anteilen von jeweils weniger als 0,1 Gew.-% enthalten und nach einer Lagerzeit von 20 Tagen bei einer Temperatur von 50°C die Anteile Cyclen D4, D5 und D6 immer noch kleiner sind als jeweils 0,1 Gew.-%, dass sie kostengünstig herstellbar sind, durch die nahezu farblosen cyclenarmen Derivate von aminofunktionellen Organopolysiloxanen weitgehend farblos sind und die mit ihnen behandelten faserigen Substrate, wie Textilien, hydrophil sind und einen weichen Griff aufweisen, der nach wiederholtem Waschen bestehen bleibt.

In den nachstehend beschriebenen Beispielen beziehen sich alle Angaben von Teilen und Prozentsätzen, falls nicht anders angegeben, auf das Gewicht. Des Weiteren beziehen sich alle Viskositätsangaben auf eine Temperatur von 25°C. Sofern nicht anders angegeben, werden die nachstehenden Beispiele bei einem Druck der umgebenden Atmosphäre, also etwa 1010 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt.

Dynamische Viskositäten wurden an einem Rheometer "MCR 302" der Fa. Anton Paar nach DIN EN ISO 3219: 1994 und DIN 93019 gemessen, wobei ein Kegel-Platte-System (Kegel CP50-2) mit einem Öffnungswinkel von 2° verwendet wurde. Die Kalibrierung des Gerätes erfolgte mit Normalöl 10000 der Physikalisch-Technischen Bundesanstalt. Die Messtemperatur betrug 25,00°C +/- 0,05°C, die Messzeit 3 min. Die Viskositätsangabe (angegeben in mPa·s) stellt den arithmetischen Mittelwert von drei unabhängig durchgeführten Einzelmessungen dar. Die Messunsicherheit der dynamischen Viskosität beträgt 1,5%. Der Schergeschwindigkeitsgradient wurde in Abhängigkeit von der Viskosität gewählt und wird für jede Viskositätsangabe separat ausgewiesen.

Die Aminzahl gibt an, wieviel mmol KOH einem Gramm der zu bestimmenden Substanz äquivalent sind. Die Bestimmung der Aminzahl erfolgt nach DIN 16945-Version 1989-03.

Die Farbzahl nach Hazen wurde nach der Methode DIN EN ISO 6271 mit dem Farbmeßgerät LICO 690 der Fa. Hach-Lange bestimmt. Die Hazen-Farbzahl (DIN-ISO 6271, auch bekannt als "APHA-Verfahren bzw. Platin-Kobalt-Skala) ist definiert als mg Platin pro 1 Liter Lösung. Für die Hazen-Stammlösung werden 1,246 g Kaliumhexachloroplatinat (IV) und 1,00 g Kobalt (II)-chlorid in 100 ml Salzsäure gelöst und mit dest. Wasser auf 1000 ml aufgefüllt. Die Hazen-Farbskala dient zur Farbbeurteilung von annähernd wasserklaren Produkten.

Zur Bestimmung der Endgruppen für die Derivate der aminofunktionellen Polydimethylsiloxane wurden $^{29}$Si-NMR-Spektren als Lösung in $C_6D_6$-Toluol an einem Bruker Avance III HD-NMR-Spektrometer (5 mm Breitbandprobenkopf mit ATMA und Z-Gradient) mit einer Messfrequenz von 90,34 MHz aufgenommen.

Die Auswertung erfolgt wie dem Fachmann bekannt und in folgender Literatur beschrieben: "Über die 1H-, 13C- und 29Si-NMR chemischen Verschiebungen einiger linearer, verzweigter und cyclischer Methyl-Siloxan-Verbindungen", G. Engelhardt, H. Jancke; J. Organometal. Chem. 28 (1971), 293-300; "Chapter 8 NMR spectroscopy of organosilicon compounds", Elizabeth A. Williams, The Chemistry of Organic Silicon Compounds, 1989 John Wiley and Sons Ltd, 511-533.

Die Bestimmung der Cyclen D4, D5 und D6 erfolgte mittels Gaschromatographie am Gerät Agilent 7890 von der Fa. Agilent Technolgies, Wilmington USA. Die Analyse erfolgte mit COC-Injektor (Cool On-Column) in eine 60m MXT5 Metall-Kapillare gekoppelt mit 5 m deaktivierter Quarz Vorsäule, und wurde am FID detektiert. Die Injektortemperatur betrug 50°C. Mit der Cool-on-Column Methode wird sichergestellt, dass keine Cyclen aus dem polymeren Organosiloxan während der Messung abgespalten werden.

Die Bestimmung des Festgehalts der Emulsionen erfolgte mittels eines Microwellenherdes von der Fa. MLS-GmbH bei einer Gesamtbestrahlungszeit von 5 Minuten und einer Bestrahlungsleistung von 650 Watt, wobei 500 mg Emulsion auf einem Glasfaserpapier vor und nach Bestrahlung gewogen wurden. Das Restgewicht wird prozentual bezüglich Einwaage angegeben.

Die Prüfung des pH-Wertes der Emulsionen erfolgte mit Indikatorstäbchen Typ Spezialindikator, pH-Bereich 2,0 - 9,0; Art.Nr. 1.09584.0001 von Merck KGaA Darmstadt.

Die Trübung der Emulsionen wurde mit dem Labor-Trübungsmesser LabScat 2 der Fa. Sigrist bestimmt. Gemessen wurde der Streuanteil von Licht aus einer 650 nm LED Lichtquelle im Winkel von 25° zum Lichtstrahl, bedingt durch die Partikel der Emulsion. Der Wert wird in ppm $SiO_2$ angegeben und entspricht einer Trübung, die diese Konzentration an Kieselgur in Wasser bewirken würde.

**Beispiel zur Aufreinigung eines aminofunktionellen Polydimethylsiloxans zur Verwendung in den Beispielen 2 bis 7**

5000 g eines nicht aufgereinigten silanol- und methoxy-terminierten seitenständig aminoethylaminopropylfunktonellen Polydimethylsiloxans von einer Viskosität von 823 mPa·s und einem Amingehalt von 0,288 mEquiv./g wurde mit einer Einfördergeschwindigkeit von 400 g/h im Laborkurzwegdestillator KDL 1 von der Fa. UIC bei einer Temperatur von 180°C und einem Druck von 0,03 mbar ausgeheizt. Hierbei bildete sich eine Destillatmenge von 52 g. Das Sumpfmaterial hatte eine Viskosität von 973 mPa·s und einen Amingehalt von 0,291 mEquiv./g. Dieses wurde als Rohstoff für die cyclenarmen Derivate von aminofunktionellen Polydimethylsiloxanen in den Beispielen 2 bis 7 verwendet.

**Beispiel 1 (Herstellung eines monohydroxy-amido-aminofunktionellen Polydimethylsiloxans mit Butyldiglykol, erfindungsgemäß)**

In einem 1000 ml 3 Hals-Kolben mit Thermoelement, KPG-Rührer und Rückflusskühler wurden 500 g eines nicht aufgereinigten silanol- und methoxy-terminierten seitenständig aminoethylaminopropylfunktonellen Polydimethylsiloxans von einer Viskosität von 939 mPa·s und einem Amingehalt von 0,288 mEquiv./g (144 mmol Amin) vorgelegt und mit Stickstoff überlagert. Bei Raumtemperatur wurden 6,49 g (75 mmol) $\gamma$-Butyrolacton, erhältlich von der Fa. Imhoff & Stahl GmbH in Mannheim, unter Rühren zugegeben. Anschließend wurde die klare Reaktionsmischung auf 100°C aufgeheizt und 5 Stunden bei dieser Temperatur gerührt. Die Aminzahl der Reaktionsmischung verringerte sich auf 0,155 mEquiv./g. Die Reaktionsmischung wurde mit einer Einfördergeschwindigkeit von 250 g/h im Laborkurzwegdestillator KDL 1 von der Fa. UIC bei einer Temperatur von 180°C und einem Druck von 0,03 mbar ausgeheizt. Hierbei bildete sich eine Destillatmenge von 5,15 g. Das Sumpfmaterial wurde in eine 3-Halskolbenrührapparatur rückgeführt und unter Rühren abgekühlt. Bei einer Temperatur von 130°C wurden 25,5 g Diethylenglykolmonobutylether, erhältlich von der Fa. Stockmeier Chemie GmbH in Bielefeld, zudosiert und langsam über 2 h auf Raumtemperatur abgekühlt. Es wurde ein fast farbloses Öl erhalten, das eine Viskosität von 2592 mPa·s, einen Restamingehalt von 0,147 mEquiv./g und eine Hazenfarbzahl von 3 aufwies. Die Cyclenanteile betrugen gemäß GC-Bestimmung (Cool-on-Column Methode) 123 ppm an D4, 96 ppm an D5 und 148 ppm an D6. Nach 20-tägiger Lagerung bei 50°C stieg der Wert von D4 auf 164 ppm an. Im Vergleich zur aminofunktionellen Vorstufe hat sich für das derivatisierte Produkt ein neues Signal im $^{29}$Si-NMR bei einer chemischen Verschiebung von -12,9 ppm gebildet, das dem terminalen diethylenglykolmonobutylether-gebundenen Siliziumatom zuzuordnen ist.

**Beispiel 2 (Herstellung eines monohydroxy-carbamato-aminofunktionellen Polydimethylsiloxans mit Butyldiglykol, erfindungsgemäß)**

In einem 1000 ml 3 Hals-Kolben mit Thermoelement, KPG-Rührer und Rückflusskühler wurden 600 g (184 mmol Amin) eines cyclenarmen silanol- und methoxy-terminierten seitenständig aminoethylaminopropylfunktonellen Polydimethylsiloxans aus dem Aufreinigungsbeispiel mit einer Viskosität von 959 mPa·s und einem Amingehalt von 0,306 mEquiv./g vorgelegt und mit Stickstoff überlagert. Die Cyclenanteile betrugen gemäß GC-Bestimmung (Cool-on-Column Methode) 230 ppm an D4, 330 ppm an D5 und 380 ppm an D6. Bei Raumtemperatur wurden 30,0 g Diethylenglykolmonobutylether und 9,38 g (92 mmol) Propylencarbonat, erhältlich von der Fa. Huntsman Holland BV in Rotterdam, unter Rühren zugegeben. Anschließend wurde die klare Reaktionsmischung auf 80°C aufgeheizt und 3 Stunden bei dieser Temperatur gerührt. Es wurde ein fast farbloses Öl erhalten, das eine Viskosität von 1907 mPa·s, einen Restamingehalt von 0,153 mEquiv./g und eine Hazenfarbzahl von 2 aufwies. Die Cyclenanteile betrugen gemäß GC-Bestimmung (Cool-on-Column Methode) 286 ppm an D4, 330 ppm an D5 und 283 ppm an D6. Nach 20-tägiger Lagerung bei 50°C stieg der Wert von D4 auf 323 ppm an. Im Vergleich zur aminofunktionellen Vorstufe hat sich für das derivatisierte Produkt ein neues Signal im $^{29}$Si-NMR bei einer chemischen Verschiebung von -12,9 ppm gebildet, das dem terminalen diethylenglykolmonobutylether-gebundenen Siliziumatom zuzuordnen ist.

**Beispiel 3 (Herstellung eines monohydroxy-carbamato-aminofunktionellen Polydimethylsiloxans mit Butyldiglykol, erfindungsgemäß)**

In einem 1000 ml 3 Hals-Kolben mit Thermoelement, KPG-Rührer und Rückflusskühler wurden 600 g (184 mmol Amin) der aminofunktionellen Polydimethylsiloxan-Vorstufe aus Beispiel 2 vorgelegt und mit Stickstoff überlagert. Bei Raumtemperatur wurden 9,38 g (92 mmol) Propylencarbonat unter Rühren zugegeben. Anschließend wurde die Reaktionsmischung auf 80°C aufgeheizt. Die zunächst trübe Mischung klarte bei Erreichen von 80°C auf und wurde 3 Stunden bei dieser Temperatur gerührt. Danach wurden 30,0 g Diethylenglykolmonobutylether eingerührt und die Mischung langsam innerhalb von 2 Stunden auf Raumtemperatur abgekühlt. Es wurde ein fast farbloses Öl erhalten, das eine Viskosität von 1278 mPa·s, einen Restamingehalt von 0,160 mEquiv./g und eine Hazenfarbzahl von 3 aufwies. Die Cyclenanteile betrugen gemäß GC-Bestimmung (Cool-on-Column Methode) 108 ppm an D4, 77 ppm an D5 und 124 ppm an D6. Nach 20-tägiger Lagerung bei 50°C stieg der Wert von D4 auf 146 ppm an. Im Vergleich zur aminofunktionellen Vorstufe hat sich für das derivatisierte Produkt ein neues Signal im $^{29}$Si-NMR bei einer chemischen Verschiebung von -12,9 ppm gebildet, das dem terminalen diethylenglykolmonobutylether-gebundenen Siliziumatom zuzuordnen ist.

**Beispiel 4 (Herstellung eines acetamido-aminofunktionellen Polydimethylsiloxans mit Butyldiglykol, nicht erfindungsgemäß)**

In einem 2000 ml 3 Hals-Kolben mit Thermoelement, KPG-Rührer, Tropftrichter und Rückflusskühler wurden 935,58 g (286 mmol Amin) der aminofunktionellen Polydimethylsiloxan-Vorstufe aus Beispiel 2 vorgelegt, mit Stickstoff überlagert und auf 55°C aufgeheizt. Dann wurden 25,17 g Diethylenglykolmonobutylether eingerührt und 30 Minuten bei 55°C weitergerührt. Daraufhin wurden 14,08 g (138 mmol) Essigsäureanhydrid, erhältlich von der Fa. Sigma-Aldrich Chemie GmbH in Steinheim, unter Rühren langsam zugetropft. Durch die Exothermie stieg die Temperatur auf 65°C an. Anschließend wurde die klare Reaktionsmischung auf 80°C aufgeheizt und 30 Minuten bei dieser Temperatur gerührt. Danach wurden noch einmal 25,17 g Diethylenglykolmonobutylether eingerührt und für eine Stunde bei 80°C nachgerührt. Es wurde ein gelbes Öl erhalten, das eine Viskosität von 2672 mPa·s, einen Restamingehalt von 0,152 mEquiv./g und eine Hazenfarbzahl von 9 aufwies. Der Cyclenanteil D4 betrug gemäß GC-Bestimmung (Cool-on-Column Methode) 148 ppm. Nach 20 Tagen Lagerung bei 50°C stieg der Cyclenanteil auf 1312 ppm an.

**Beispiel 5 (Herstellung eines pentahydroxy-amido-aminofunktionellen Polydimethylsiloxans mit Butyldiglykol, nicht erfindungsgemäß)**

In einem 500 ml 3 Hals-Kolben mit Thermoelement, KPG-Rührer, Tropftrichter, Rückflusskühler, Destillationsbrücke und Vakuumpupe mit Regelung wurden 180 g (55 mmol Amin) der aminofunktionellen Polydimethylsiloxan-Vorstufe aus Beispiel 2 vorgelegt zusammen mit 180 g Isopropanol vorgelegt und mit Stickstoff überlagert. Bei Raumtemperatur und Normaldruck wurden unter Rühren 4,73 g (55 mmol) Gluconolacton, erhältlich von der Fa. Sigma-Aldrich Chemie GmbH in Steinheim, unter Rühren zugegeben. Anschließend wurde die trübe Reaktionsmischung auf 80°C aufgeheizt. Die Mischung wurde 3 Stunden bei dieser Temperatur gerührt klarte dabei zunehmend auf. Danach wurde bei dieser Temperatureinstellung ein Vakuum von 350 mbar angelegt und Isopropanol abdestilliert. Gleichzeit wurden über den Tropftrichter 16,2 g Diethylenglykolmonobutylether eingerührt und das Vakuum über 2 Stunden auf 180 mbar verbessert. Die Temperatur der Mischung verringerte sich dabei, je nach Destillationsrate, auf 65° bis 55°C. Es wurden 160 g Destillat erhalten. Der Kolbeninhalt wurde zunehmend zäh und gelblich mit leichter Trübung. Es wurde ein homogenes, zähfließendes und gelbliches Öl erhalten, das eine Viskosität von 7180 mPa·s, einen Restamingehalt von 0,075 mEquiv./g und eine Hazenfarbzahl von 9 aufwies.

**Beispiel 6 (Herstellung eines dihydroxy-carbamato-aminofunktionellen Polydimethylsiloxans mit Butyldiglykol, nicht erfindungsgemäß)**

In einem 1000 ml 3 Hals-Kolben mit Thermoelement, KPG-Rührer und Rückflusskühler wurden 500 g (153 mmol Amin) der aminofunktionellen Polydimethylsiloxan-Vorstufe aus Beispiel 2 vorgelegt und mit Stickstoff überlagert. Bei Raumtemperatur wurden 25,0 g Diethylenglykolmonobutylether und 8,71 g (74 mmol) Glycerincarbonat, erhältlich von der Fa. Huntsman Holland BV in Rotterdam, unter Rühren zugegeben. Anschließend wurde die trübe Reaktionsmischung auf 80°C aufgeheizt und 3 Stunden bei dieser Temperatur gerührt. Es wurde ein klares, leicht gelbliches, zähfließendes Öl erhalten, das eine Viskosität von 17800 mPa·s, einen Restamingehalt von 0,152 mEquiv./g und eine Hazenfarbzahl von 5 aufwies.

**Beispiel 7 (Herstellung eines monohydroxy-carbamato-aminofunktionellen Polydimethylsiloxans mit Isotridecanol, nicht erfindungsgemäß)**

In einem 500 ml 3 Hals-Kolben mit Thermoelement, KPG-Rührer und Rückflusskühler wurden 300 g (92 mmol Amin) der aminofunktionellen Polydimethylsiloxan-Vorstufe aus Beispiel 2 vorgelegt und mit Stickstoff überlagert. Bei Raumtemperatur wurden 15,0 g Isotridecanol (erhältlich von ABCR GmbH, Karlsruhe) und 4,69 g (46 mmol) Propylencarbonat unter Rühren zugegeben. Anschließend wurde die Reaktionsmischung auf 80°C aufgeheizt und 3 Stunden bei dieser Temperatur gerührt. Der Kolbeninhalt klarte bei Erreichen von 75°C auf. Es wurde ein fast farbloses zähfließendes Öl erhalten, das eine Viskosität von 9500 mPa·s und einen Restamingehalt von 0,176 mEquiv./g aufwies. Im Vergleich zur aminofunktionellen Vorstufe haben sich für das derivatisierte Produkt mehrere neue Signale im $^{29}$Si-NMR

bei einer chemischen Verschiebung im Bereich von -13,8 ppm gebildet, die den terminalen isotridecanol-gebundenen Siliziumatomen zuzuordnen sind.

**Beispiel 8 (Herstellung einer Emulsion aus dem erfindungsgemäßen monohydroxy-amido-aminofunktionellen Polydimethylsiloxan aus Beispiel 1)**

21,0 g vollentsalztes Wasser, 12,0 g Tridecylalkoholethoxylat mit 5 EO, 8,5 g Tridecylalkoholethoxylat mit 8 EO (erhältlich als LUTENSOL® TO 5 bzw. LUTENSOL® TO 8 von BASF SE, Ludwigshafen), 4,0 g Diethylenglykolmonobutylether und 2,0 g Laureth-11-carbonsäure (erhältlich als Akypo RLM 100 von Kao Chemicals GmbH, Emmerich) wurden am Labordissolver (Dispermat CN 30 von der Fa. VMA-Getzmann GmbH, Reichshof) bei einer Drehzahl von 800 UpM gemischt. Anschließend wurden 35,0 g des monohydroxy-amido-aminofunktionellen Polydimethylsiloxan aus Beispiel 1 bei einer Drehzahl von 1000 UpM in drei etwa gleichen Portionen eingearbeitet. Die dicke cremige Mischung wurde zunächst mit 4,87 g und dann mit 12,5 g vollentsalztem Wasser verdünnt. Die fast farblose Microemulsion hatte einen Festgehalt von 62%, einen pH von 6,0 und einen Trübungswert von 4 ppm $SiO_2$.

**Beispiel 9 (Herstellung einer Emulsion aus dem erfindungsgemäßen monohydroxy-carbamato-aminofunktionellen Polydimethylsiloxan aus Beispiel 2)**

35,0 g des monohydroxy-carbamato-aminofunktionellen Polydimethylsiloxans aus Beispiel 2 wurden analog Beispiel 8 zu einer fast farblosen Microemulsion mit einem Festgehalt von 61%, einen pH von 6,0 und einen Trübungswert von 1 ppm $SiO_2$ emulgiert.

**Beispiel 10 (Herstellung einer Emulsion aus dem nicht erfindungsgemäßen acetamido-aminofunktionellen Polydimethylsiloxan aus Beispiel 4)**

35,0 g des acetamido-aminofunktionellen Polydimethylsiloxans aus Beispiel 4 wurden analog Beispiel 8 zu einer gelben Microemulsion mit einem Festgehalt von 61%, einen pH von 4,5 und einen Trübungswert von 3 ppm SiOz emulgiert.

**Beispiel 11 (Herstellung einer Emulsion aus dem nicht erfindungsgemäßen pentahydroxy-amido-aminofunktionellen Polydimethylsiloxans aus Beispiel 5)**

21,0 g vollentsalztes Wasser, 12,0 g Tridecylalkoholethoxylat mit 5 EO, 8,5 g Tridecylalkoholethoxylat mit 8 EO und 2,0 g Laureth-11-carbonsäure (erhältlich als Akypo RLM 100 von Kao Chemicals GmbH, Emmerich) wurden am Labordissolver bei einer Drehzahl von 800 UpM gemischt. Anschließend wurden 35,0 g des pentahydroxy-amido-aminofunktionellen Polydimethylsiloxan aus Beispiel 5 bei einer Drehzahl von 1000 UpM in drei etwa gleichen Portionen eingearbeitet. Die stark viskose Mischung wurde zunächst mit 4,87 g und dann mit 12,5 g vollentsalztem Wasser verdünnt. Die trübe, viskose Emulsion hatte einen Festgehalt von 62%, einen pH von 5,5 und einen Trübungswert von 250 ppm $SiO_2$. Der fehlende Anteil an Diethylenglykolmonobutylether wurde durch dessen erhöhten Anteil im pentahydroxy-amido-aminofunktionellen Polydimethylsiloxan ausgeglichen.

**Beispiel 12 (Herstellung einer Emulsion aus dem nicht erfindungsgemäßen dihydroxy-carbamato-aminofunktionellen Polydimethylsiloxan aus Beispiel 6)**

35,0 g des dihydroxy-carbamato-aminofunktionellen Polydimethylsiloxans aus Beispiel 6 wurden analog Beispiel 8 zu einer trüben Emulsion mit einem Festgehalt von 60%, einen pH von 6,5 und einen Trübungswert von 140 ppm $SiO_2$ emulgiert.

**Beispiel 13 (Herstellung einer Emulsion aus dem nicht erfindungsgemäßen monohydroxy-carbamato-aminofunktionellen Polydimethylsiloxan aus Beispiel 7)**

35,0 g des monohydroxy-carbamato-aminofunktionellen Polydimethylsiloxans aus Beispiel 7 wurden analog Beispiel 8 zu einer fast farblosen, aber diffus trüben Emulsion mit einem Festgehalt von 62%, einen pH von 5,5 und einen Trübungswert von 253 ppm $SiO_2$ emulgiert. Nach 3 Wochen Lagerung setzte eine Trennung in 2 Phasen ein.

**Beispiel 14 (Herstellung einer Emulsion aus dem aminofunktionellen Polydimethylsiloxan, Rohstoff aus den Beispielen 2 bis 7; Weichgriffreferenz)**

21,0 g vollentsalztes Wasser, 12,0 g Tridecylalkoholethoxylat mit 5 EO, 8,5 g Tridecylalkoholethoxylat mit 8 EO, 4,0 g Diethylenglykolmonobutylether, 2,0 g Laureth-11-carbonsäure und 0,32 g Eisessig wurden am Labordissolver bei einer Drehzahl von 800 UpM gemischt. Anschließend wurden 35,0 g des cyclenarmen silanol- und methoxy-terminierten seitenständig aminoethylaminopropylfunktonellen Polydimethylsiloxan-Rohstoffs von einer Viskosität von 959 mPa·s und einem Amingehalt von 0,306 mEquiv./g aus den Beispielen 2 bis 7 bei einer Drehzahl von 1000 UpM in drei etwa gleichen Portionen eingearbeitet. Die dicke cremige Mischung wurde zunächst mit 4,87 g und dann mit 12,5 g vollentsalztem Wasser verdünnt. Die farblose, opaleszierende Microemulsion hatte einen Festgehalt von 61%, einen pH von 5,0 und einen Trübungswert von 42 ppm SiOz.

**Beispiel 15 (Herstellung von eines Silicon-Polyether Copolymers analog Beispiel 4 in US 2008/0075683 A1 für hydrophile Referenzemulsion)**

635 g eines $\alpha,\omega$-Dihydrogenpolydimethylsiloxans mit 0,052 Gew.-% Si-gebundenem Wasserstoff wurden mit 205 g eines Polyethers der Formel $H_2C{=}CH{-}CH_2{-}(OCH_2CH_2)_{9,5}{-}OH$ gemischt. Die Mischung wurde auf 100°C erwärmt und zur Mischung werden 0,28 g einer 2,7 Gew.-%igen (bezogen auf elementares Platin) Lösung eines Platin-1,3-Divinyl-1,1,3,3-tetramethyldisiloxan-Komplexes in einem $\alpha,\omega$ -Divinyldimethylpolysiloxan mit einer Viskosität von 1000 mPa·s bei 25°C, eine Lösung des sogenannten Karstedt-Katalysators (dessen Herstellung in US 3,775,452 beschrieben ist), zudosiert, worauf die Temperatur des Reaktionsgemisches um 19°C anstieg und ein klares Produkt entstand. Nach einer Stunde bei 100 bis 110°C wurde vollständiger Umsatz des Sigebundenen Wasserstoffs erreicht. Das Polyether-Polysiloxan-Zwischenprodukt hatte eine OH-Konzentration von 0,512 mEquiv./g und enthielt 177 ppm Wasser. 200 g dieses Zwischenprodukts wurden mit 10,3 g Bis(dimethylaminopropyl)amin gemischt und auf 84°C erwärmt; 13,2 g Hexamethylendiisocyanat wurden zudosiert.

Das Verhältnis der NCO-Gruppen zur Summe der damit reagierenden organischen Funktionen betrug 0,998, bei Einbeziehung von Wasser 0,97.

Ohne weitere Katalyse wurde in einer Stunde bei ca. 90°C in leicht exothermer Reaktion ein kompletter Umsatz der Isocyanatgruppen erreicht. Das Polymergemisch enthielt 0,49 mEquiv./g basischen Stickstoff. 32 g dieses Polymers wurden mit einer Lösung von 1,04 g Essigsäure in 8 g Diethylenglycolmonobutylether neutralisiert.

**Beispiel 16 (Herstellung einer Emulsion aus dem Silicon-Polyether Copolymer in Beispiel 15; Hydrophiliereferenz)**

21,0 g vollentsalztes Wasser, 12,0 g Tridecylalkoholethoxylat mit 5 EO, 8,5 g Tridecylalkoholethoxylat mit 8 EO und 2,0 g Laureth-11-carbonsäure wurden am Labordissolver bei einer Drehzahl von 800 UpM gemischt. Anschließend wurden 35,0 g des Silicon-Polyether Copolymers in Beispiel 15 bei einer Drehzahl von 1000 UpM in 3 etwa gleichen Portionen eingearbeitet. Die fließfähige Mischung wurde zunächst mit 4,87 g und dann mit 12,5 g vollentsalztem Wasser verdünnt. Die milchige Emulsion hatte einen Festgehalt von 54% und einen pH von 6,0.

**Anwendungstechnische Tests:**

Zur Textilausrüstung wurde eine unausgerüstete Maschenware 100% CO Interlock mit einem Flächengewicht von 190 $g/m^2$ verwendet.

Als Referenz diente eine mit Wasser geklotzte und getrocknete Ware (= Blindwert).

Der Stoff wurde mit der jeweiligen Flotte getränkt, mit einem Zweiwalzenfoulard auf eine Flottenaufnahme von 77% abgequetscht, aufgespannt und die Wirkware in einem MATHIS-Laborspannrahmen bei 150°C drei Minuten getrocknet. Anschließend wurde die Ware mindestens 12 Stunden im Klimaraum bei 23°C und 62 % Luftfeuchtigkeit klimatisiert.

Bestimmungsmethoden für die Ergebnisse der Anwendungsbeispiele:

*Bestimmung des Weichgriffs (Griffbewertung):*

Da der Weichgriff von Textilien stark dem subjektiven Empfinden der Testpersonen unterliegt, kann nur eine Standardisierung der Randbedingungen, nicht aber der Bewertung erreicht werden. Um trotzdem eine Reproduzierbarkeit zu gewährleisten, wurden die ausgerüsteten Muster hinsichtlich ihres Weichgriffs beurteilt und in eine Rangfolge gebracht. Dazu wurden von vier Personen in Abhängigkeit der Anzahl der getesteten Muster Punkte vergeben, wobei die Höhe der Punktzahl mit dem Weichgriff korreliert. Das weichste Muster erhält die maximale Punktzahl, während das

am wenigsten weiche Muster 0 Punkte erhält. Die Griffbewertung eines Musters errechnet sich somit als Mittelwert der jeweils auf dieses Muster entfallenen Punkte.

*Bestimmung der Tropfeneinsinkzeit:*

Das ausgerüstete Muster wurde nach der Applikation des Siliconproduktes acht Stunden zur Akklimatisierung in einem Klimaraum bei einer Temperatur von 23°C und einer Luftfeuchtigkeit von 62% gelagert, dann wurde ein Tropfen entionisiertes Wasser aus einer Höhe von 1 cm auf die gespannte Stoffoberfläche gegeben und die Zeit bestimmt, nach der der Wassertropfen vom Stoff aufgesaugt war, längstens jedoch drei Minuten (180 Sekunden). Es wurden fünf Bestimmungen durchgeführt und der Mittelwert gebildet.

*Bestimmung der Waschfestigkeit:*

Für die Untersuchung der Waschechtheiten wurden alle ausgerüsteten Textilien zusammen mit ca. 3 kg Ballaststoff in einer Haushaltswaschmaschine der Marke SIWAMAT 6143 von Siemens mit dem Buntwäscheprogramm bei 60°C gewaschen und bei 1400 UpM geschleudert. Als Waschtensid wurden dabei 36 g eines Flüssigwaschmittels der Marke "Spee Feincolor" von der Firma Henkel dosiert. Es wurden insgesamt 2 Waschzyklen von jeweils 90 Minuten Dauer ohne Zwischentrocknung durchgeführt. Anschließend wurde die Ware mindestens 12 Stunden im Klimaraum bei 23°C und 62 % Luftfeuchtigkeit getrocknet und klimatisiert. Die Warenmuster wurden dann erneut einem Weichgriffvergleich unterzogen.

In der folgenden Tabelle sind für einige Anwendungsbeispiele die Ergebnisse des mittels Foulardverfahren ausgerüsteten Stoffs zusammengestellt.

Tabelle:

| Beispiele und Vergleich | Festgehalt in [%] | Einsatzmenge in [g/l] | Tropfentest in [s] | Weichgriff vor dem Waschen | Weichgriff nach 2 Wäschen |
|---|---|---|---|---|---|
| Beispiel 8 | 62 | 10,0 | 4 | 4,75 | 5,13 |
| Beispiel 9 | 61 | 10,0 | 3 | 5,25 | 5, 62 |
| Beispiel 10 (Vergleich) | 61 | 10,0 | 17 | 4,25 | 4, 63 |
| Beispiel 11 (Vergleich) | 62 | 10,0 | 28 | 2,88 | 3,50 |
| Beispiel 12 (Vergleich) | 60 | 10,0 | 16 | 3,25 | 3,87 |
| Beispiel 13 (Vergleich) | 62 | 10,0 | 9 | 3, 62 | 4,25 |
| Beispiel 14 (Griffreferenz) | 61 | 10,0 | 85 | 7,75 | 8 |
| Beispiel 16 (Hydrophiliereferenz) | 54 | 11,3 | 1 | 4,25 | 1 |
| Blindwert | | | 0 | 0 | 0 |

Die erfindungsgemäß mit monohydroxy-amido- bzw. monohydroxy-carbamato-haltigen aminofunktionellen Polydimethylsiloxanen (Derivatisierung mit γ-Butyrolacton bzw. Propylencarbonat, Beispiele 8 bzw. 9) ausgerüsteten Textilien zeigen im Vergleich zu mit acetamido-, pentahydroxy-amido- und dihydroxy-carbamatohaltigen aminofunktionellen Polydimethylsiloxanen (Derivatisierung mit Essigsäureanhydrid, Gluconolacton bzw. Glycerincarbonat, Vergl.-Beispiele 10, 11 und 12) sowie mit nicht derivatisierten aminofunktionellen Polydimethylsiloxanen (Vergl.-Beispiel 14) ausgerüsteten Textilien eine deutlich kürzere Tropfeneinsinkzeit. Der Fachmann würde erwarten, dass, durch höheren Anteil an Hydroxygruppen, polarere funktionelle Gruppen die faserigen Substrate mit hydrophileren Eigenschaften ausstatten sollten. Überraschenderweise zeigen sich jedoch die erfindungsgemäßen monohydroxyhaltigen Derivate am hydrophilsten in der Gruppe der nicht polyetherhaltigen aminofunktionellen Polydimethylsiloxane. Die Emulsion des erfindungsgemäßen mit Diethylenglykolmonobutylether terminierten monohydroxy-carbamato-haltigen aminofunktionellen

Polydimethylsiloxans (Beispiel 9) hat neben dem klareren Aussehen und der besseren Stabilität auch Vorteile in der Hydrophilie gegenüber der vergleichbaren, aber nicht erfindungsgemäßen Emulsion aus dem mit Isotridecanol terminierten Polydimethylsiloxan-Öl (Vergl.-Beispiel 13). Im Vergleich zum über Hydrosilylierung und Isocyanatverbrückung hergestellten Silicon-Polyether-Copolymer (Vergl.-Beispiel 16) präsentieren sich die erfindungsgemäßen monohydroxyhaltigen Aminosilicon-Derivate (Beispiele 8 und 9) bezüglich des Weichgriffs als sehr waschbeständig, obwohl sie sich durch die guten hydrophilen Eigenschaften auszeichnen. Die rein aminofunktionelle Griffreferenz (Vergl.-Beispiel 14) hat zwar sehr gute Weichgriffeigenschaften sowohl vor als auch nach der Wäsche, jedoch kann sie nicht in der hydrophilen Ausrüstung eingesetzt werden.

Wie die Beispiele 1 bis 4 zeigen, bilden im Gegensatz zum nicht erfindungsgemäßen acetamido-haltigen Aminosilicon-Derivat (Vergl.-Beispiele 4) die erfindungsgemäßen monohydroxy-haltigen Aminosilicon-Derivate der Beispiele 1 bis 3 in der anspruchsvollen Lagerung bei 50°C keine höheren Mengen an Cyclen D4 nach, so dass von einer guten Lagerstabilität im Hinblick auf cyclenarme Eigenschaften und den damit nicht erforderlichen SVHC-Einstufungen (substances of very high concern) ausgegangen werden kann. Die Viskositäten der erfindungsgemäßen Derivate aminofunktioneller Polydimethylsiloxane liegen alle im Bereich unterhalb von 5000 mPa·s bei 25°C.

**Patentansprüche**

1. Verwendung von Zusammensetzungen zur hydrophilen und im Weichgriff waschbeständigen Ausrüstung von faserigen Substraten enthaltend Derivate von aminofunktionellen Organopolysiloxanen enthaltend Siloxaneinheiten der allgemeinen Formel(I)

$$R_aZ_bSiO_{\frac{4-(a+b)}{2}} \qquad (I),$$

wobei

a 0, 1 oder 2, vorzugsweise 0 oder 1, ist
b 1, 2 oder 3, vorzugsweise 1, ist
mit der Maßgabe, dass die Summe aus a+b ≤ 3 ist,
gegebenenfalls Siloxaneinheiten der allgemeinen Formel (II)

$$R_cQ_dSiO_{\frac{4-(c+d)}{2}} \qquad (II),$$

wobei
c 0, 1 oder 2, vorzugsweise 0 oder 1, ist
d 1, 2 oder 3, vorzugsweise 1, ist
mit der Maßgabe, dass die Summe aus c+d ≤ 3 ist,
Siloxaneinheiten der allgemeinen Formel (III)

$$R_eSiO_{\frac{4-e}{2}} \qquad (III),$$

wobei
e 0, 1, 2, oder 3, vorzugsweise 2 ist,
und Siloxaneinheiten der allgemeinen Formel (IV)

$$Y_fR_gSiO_{\frac{4-(f+g)}{2}} \qquad (IV),$$

wobei
f 1, 2 oder 3, vorzugsweise 1, ist,
g 0, 1 oder 2, vorzugsweise 2, ist,

mit der Maßgabe, dass die Summe aus $f+g \leq 3$ ist,
R gleich oder verschieden sein kann, und ein Wasserstoffatom oder einen einwertigen, gegebenenfalls Fluor-, Chlor- oder Brom-substituierten $C_1$- bis $C_{18}$-Kohlenwasserstoffrest bedeutet,
Z eine Gruppe der allgemeinen Formel (V) bedeutet

$$-R^2-[NR^3(CH_2)_n]_iNR^4R^5 \quad (V),$$

wobei
i 0, 1, 2, 3 oder 4 ist,
n 2, 3, 4, 5 oder 6,
$R^2$ einen zweiwertigen, linearen oder verzweigten $C_1$- bis $C_{18}$-Kohlenwasserstoffrest bedeutet,
$R^3$ ein Wasserstoffatom, einen gegebenenfalls Fluor-, Chlor-, Brom-, Hydroxy- oder $C_1$- bis $C_5$-alkoxysubstituierten $C_1$- bis $C_{18}$-Kohlenwasserstoffrest, oder einen Acylrest oder einen Rest der allgemeinen Formel (VI) bedeutet,
$R^4$ einen Rest der allgemeinen Formel (VI) bedeutet

$$-(C{=}O)-R^6-OH \quad (VI),$$

$R^5$ ein Wasserstoffatom oder einen gegebenenfalls Fluor-, Chlor-, Brom-, Hydroxy- oder $C_1$- bis $C_5$-alkoxysubstituierten $C_1$- bis $C_{18}$-Kohlenwasserstoffrest oder einen Acylrest bedeutet,
$R^6$ einen zweiwertigen, linearen oder verzweigten $C_2$- bis $C_8$-Kohlenwasserstoffrest oder einen Rest $-OR^7$-bedeutet,
$R^7$ einen zweiwertigen linearen oder verzweigten $C_2$ bis $C_8$-Kohlenwasserstoffrest bedeutet,
Q eine Gruppe der allgemeinen Formel (VII) bedeutet

$$-R^2-[NR^9(CH_2)_n]_iN(R^9)_2 \quad (VII),$$

wobei i und n die oben dafür angegebene Bedeutung haben, $R^9$ gleich oder verschieden sein kann, und ein Wasserstoffatom, einen gegebenenfalls Fluor-, Chlor-, Brom-, Hydroxy-oder $C_1$- bis $C_5$-alkoxysubstituierten $C_1$- bis $C_{18}$-Kohlenwasserstoffrest oder einen Acylrest bedeutet,
Y einen Rest der allgemeinen Formel (VIII) und/oder (IX) bedeutet

$$-OR^1 \text{ (VIII) und/oder } -[O(CHR^{10})_p]_mOR^{11} \quad (IX),$$

wobei
m 0 oder eine ganze Zahl von 1 bis 100, vorzugsweise 1 bis 20, ist,
p 2, 3 oder 4 ist,
$R^1$ ein Wasserstoffatom oder einen $C_1$- bis $C_4$-Alkylrest bedeutet,
$R^{10}$ ein Wasserstoffatom oder einen $C_1$- bis $C_{18}$-Kohlenwasserstoffrest bedeutet,
$R^{11}$ ein Wasserstoffatom, einen $C_1$- bis $C_{10}$-Kohlenwasserstoffrest, vorzugsweise einen $C_4$- bis $C_{10}$-Kohlenwasserstoffrest, oder eine Gruppe der Formel $-(C{=}O)-R^{12}$ bedeutet,
$R^{12}$ einen $C_1$- bis $C_{10}$-Kohlenwasserstoffrest oder einen Rest $-O-R^{13}$ bedeutet, und
$R^{13}$ einen $C_1$- bis $C_{10}$-Kohlenwasserstoffrest bedeutet,
mit der Maßgabe, dass mindestens ein Teil der Reste Y Reste der Formel (IX) sind,
mit der Maßgabe, dass Verunreinigungen an Octamethylcyclotetrasiloxan (Cyclen D4), Decamethylcyclopentasiloxan (Cyclen D5) und Dodecamethylcyclohexasiloxan (Cyclen D6) in Anteilen von jeweils weniger als 0,1 Gew.-% enthalten sind und nach einer Lagerzeit von 20 Tagen bei einer Temperatur von 50°C die Anteile an Cyclen D4, repräsentativ für D4, D5 und D6, immer noch kleiner sind als jeweils 0,1 Gew.-%, jeweils bezogen auf das Gesamtgewicht der Derivate von aminofunktionellen Organopolysiloxanen.

2. Verwendung nach Anspruch 1, **dadurch gekennzeichnet, dass** in den erfindungsgemäßen Zusammensetzungen Derivate von aminofunktionellen Organopolysiloxanen der allgemeinen Formel (X) eingesetzt werden

$$YR_2SiO-(R_2SiO)_k-(RZSiO)_l-(RQSiO)_o-R_2SiY \quad (X),$$

wobei

R, Y, Z und Q die in Anspruch 1 dafür angegebenen Bedeutungen haben,

k eine ganze Zahl von 50 bis 700 ist,
1 eine ganze Zahl von 1 bis 30 ist und
o eine ganze Zahl von 0 bis 30 bedeuten, bevorzugt 1 bis 15, ist,
mit der Maßgabe, dass mindestens ein Teil der Reste Y Reste der Formel (IX) sind.

3. Verwendung nach Anspruch 1 oder 2, **dadurch gekennzeichnet, dass** ein Teil der Reste Y (Iso)Oxyalkylresten der Formel (IX) entspricht, wobei es sich um $C_4$-$C_{10}$-Alkoxyreste oder $C_4$-$C_{10}$-Monoalkylglykoletherreste handelt, neben Resten der Formel (VIII), die Hydroxyl- und/oder Methoxyreste sind.

4. Verwendung nach Anspruch 1 bis 3, **dadurch gekennzeichnet, dass** als Zusammensetzungen wässrige Emulsionen enthaltend cyclenarme Derivate von aminofunktionellen Organopolysiloxanen (A) gemäß Anspruch 1, 2, oder 3, Emulgatoren (B) und/oder Coemulgatoren (B') und
Wasser (C)
eingesetzt werden.

5. Verfahren zur waschpermanenten Ausrüstung von faserigen Substraten nach einem der Ansprüche 1 bis 4, **dadurch gekennzeichnet, dass** die Zusammensetzungen auf die faserigen Substrate aufgebracht werden und die so behandelten faserigen Substrate bei Temperaturen von vorzugsweise 20 bis 200°C trocknen gelassen werden.

6. Verfahren nach Anspruch 5, **dadurch gekennzeichnet, dass** die faserigen Substrate Textilien sind.

7. Verfahren zur Herstellung der in den Zusammensetzungen eingesetzten cyclenarmen Derivate von aminofunktionellen Organopolysiloxanen nach Anspruch 1 bis 4, indem aminofunktionelle Organopolysiloxane (1), enthaltend aminofunktionelle Siloxaneinheiten der allgemeinen Formel (II),

$$R_c Q_d SiO_{\frac{4-(c+d)}{2}} \quad (II),$$

wobei

Q, R, c und d die in Anspruch 1 dafür angegebene Bedeutung haben,
und Siloxaneinheiten der allgemeinen Formel (XII)

$$R_g (OR^1)_f SiO_{\frac{4-(f+g)}{2}} \quad (XII),$$

wobei
R, $R^1$, f und g die in Anspruch 1 dafür angegebene Bedeutung haben,
mit Verbindungen (2) der allgemeinen Formel (XI)

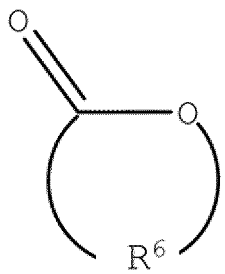
(XI),

wobei
$R^6$ die in Anspruch 1 dafür angegebenen Bedeutungen hat,
und Verbindungen (3) der allgemeinen Formel (XIII)

$$H\text{-}[O(CHR^{10})_p]_mOR^{11} \qquad (XIII),$$

wobei
$R^{10}$, $R^{11}$, m und p die in Anspruch 1 dafür angegebene Bedeutung haben, umgesetzt werden,
mit der Maßgabe, dass in den erhaltenen Derivaten von aminofunktionellen Organopolysiloxanen der Gehalt an Cyclen D4, D5 und D6 jeweils kleiner als 0,1 Gew.-% ist, indem die Cyclen vor, während oder nach der Umsetzung destillativ entfernt werden.

**8.** Verfahren nach Anspruch 7, **gekennzeichnet dadurch, dass** aminofunktionelle Organopolysiloxane (1) enthaltend aminofunktionelle Siloxaneinheiten der allgemeinen Formel (II) und Siloxaneinheiten der allgemeinen Formel (XII) mit Verbindungen (2) der allgemeinen Formel (XI) und Verbindungen (3) der allgemeinen Formel (XIII) gleichzeitig umgesetzt werden.

**9.** Verfahren nach Anspruch 7, **gekennzeichnet dadurch, dass** aminofunktionelle Organopolysiloxane (1) enthaltend aminofunktionelle Siloxaneinheiten der allgemeinen Formel (II) und Siloxaneinheiten der allgemeinen Formel (XII) in einem ersten Reaktionsschritt mit Verbindungen (2) der allgemeinen Formel (XI), und die daraus gebildeten Reaktionsprodukte anschließend in einem weiteren Reaktionsschritt mit Verbindungen (3) der allgemeinen Formel (XIII) umgesetzt werden.

**10.** Verfahren nach Anspruch 7, **gekennzeichnet dadurch, dass** aminofunktionelle Organopolysiloxane (1) enthaltend aminofunktionelle Siloxaneinheiten der allgemeinen Formel (II) und Siloxaneinheiten der allgemeinen Formel (XII) in einem ersten Reaktionsschritt mit Verbindungen (3) der allgemeinen Formel (XIII), und die daraus gebildeten Reaktionsprodukte anschließend in einem weiteren Reaktionsschritt mit Verbindungen (2) der allgemeinen Formel (XI) umgesetzt werden.

**11.** Verfahren zur Herstellung der in den Zusammensetzungen eingesetzten cyclenarmen Derivate von aminofunktionellen Organopolysiloxanen nach Anspruch 1 bis 4, indem aminofunktionelle Organopolysiloxane (1), enthaltend aminofunktionelle Siloxaneinheiten der allgemeinen Formel (II)

$$R_cQ_dSiO_{\frac{4-(c+d)}{2}} \qquad (II),$$

wobei

Q, R, c und d die in Anspruch 1 dafür angegebene Bedeutung haben,
und Siloxaneinheiten der allgemeinen Formel (XII)

$$R_g(OR^1)_fSiO_{\frac{4-(f+g)}{2}} \qquad (XII),$$

wobei
R, $R^1$, f und g die in Anspruch 1 dafür angegebene Bedeutung haben,
in einem ersten Schritt mit Verbindungen (2) der allgemeinen Formel (XI)

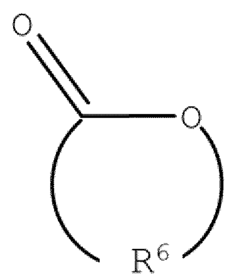
(XI),

wobei,

$R^6$ die in Anspruch 1 dafür angegebenen Bedeutungen hat, umgesetzt werden und anschließend aus dem Reaktionsprodukt Cyclen D4, D5 und D6 destillativ entfernt werden, und in einem 2. Schritt das aufgereinigte Reaktionsprodukt aus dem ersten Schritt mit Verbindungen (3) der allgemeinen Formel (XIII)

$$H\text{-}[O(CHR^{10})_p]_mOR^{11} \qquad (XIII),$$

wobei
$R^{10}$, $R^{11}$, m und p die in Anspruch 1 dafür angegebene Bedeutung haben, umgesetzt werden, mit der Maßgabe, dass in den erhaltenen Derivaten von aminofunktionellen Organopolysiloxanen der Gehalt an Cyclen D4, D5 und D6 jeweils kleiner als 0,1 Gew.-% ist.

**12.** Verfahren nach Anspruch 7 bis 11, **gekennzeichnet dadurch, dass** als aminofunktionelle Organopolysiloxane (1) solche der allgemeinen Formel (XIV) eingesetzt werden,

$$R^1OR_2SiO\text{-}(R_2SiO)_k\text{-}(RQSiO)_{l+o}\text{-}SiR_2OR^1 \qquad (XIV),$$

wobei
R und $R^1$ die in Anspruch 1 dafür angegebene Bedeutung haben und k, 1 und o die in Anspruch 2 dafür angegebene Bedeutung haben.

**13.** Verfahren nach Anspruch 7 bis 12, **dadurch gekennzeichnet, dass** als Verbindungen (2) der allgemeinen Formel (XI) Lactone oder cyclische Kohlensäureester eingesetzt werden.

**14.** Verfahren nach Anspruch 7 bis 13, **dadurch gekennzeichnet, dass** als Verbindungen (3) der allgemeinen Formel (XIII) ein $C_4$- bis $C_{10}$-Alkohol oder ein $C_4$- bis $C_{10}$-Monoalkylglykolether eingesetzt wird.

**15.** Verfahren nach Anspruch 7 bis 14, **dadurch gekennzeichnet, dass** als Verbindungen (2) der allgemeinen Formel (XI) γ-Butyrolacton oder Propylencarbonat eingesetzt werden.

## Claims

**1.** Use of compositions for hydrophilic treatment of fibrous substrates that is washfast in terms of softness containing derivatives of amino-functional organopolysiloxanes containing

siloxane units of general formula (I)

$$R_aZ_bSiO_{\frac{4-(a+b)}{2}} \qquad (I),$$

wherein
a is 0, 1 or 2, preferably 0 or 1,
b is 1, 2 or 3, preferably 1,
with the proviso that the sum of a+b ≤ 3,
optionally siloxane units of general formula (II)

$$R_cQ_dSiO_{\frac{4-(c+d)}{2}} \qquad (II),$$

wherein
c is 0, 1 or 2, preferably 0 or 1,
d is 1, 2 or 3, preferably 1,
with the proviso that the sum of c+d ≤ 3,
siloxane units of general formula (III)

$$R_eSiO_{\frac{4-e}{2}} \qquad (III),$$

wherein
e is 0, 1, 2, or 3, preferably 2,
and siloxane units of general formula (IV)

$$Y_fR_gSiO_{\frac{4-(f+g)}{2}} \qquad (IV),$$

wherein
f is 1, 2 or 3, preferably 1,
g is 0, 1 or 2, preferably 2,
with the proviso that the sum of $f+g \leq 3$,
R may be identical or different and represents a hydrogen atom or a monovalent, optionally fluorine-, chlorine- or bromine-substituted, $C_1$- to $C_{18}$-hydrocarbon radical,
Z represents a group of general formula (V)

$$-R^2-[NR^3(CH_2)_n]_iNR^4R^5 \qquad (V),$$

wherein
i is 0, 1, 2, 3 or 4,
n is 2, 3, 4, 5 or 6,
$R^2$ represents a divalent, linear or branched $C_1$- to $C_{18}$-hydrocarbon radical,
$R^3$ represents a hydrogen atom, an optionally fluorine-, chlorine-, bromine-, hydroxy- or $C_1$- to $C_5$-alkoxy-substituted $C_1$- to $C_{18}$-hydrocarbon radical, or an acyl radical or a radical of general formula (VI),
$R^4$ represents a radical of general formula (VI)

$$-(C{=}O)-R^6-OH \qquad (VI),$$

$R^5$ represents a hydrogen atom or an optionally fluorine-, chlorine-, bromine-, hydroxy- or $C_1$- to $C_5$-alkoxy-substituted $C_1$- to $C_{18}$-hydrocarbon radical or an acyl radical,
$R^6$ represents a divalent, linear or branched $C_2$- to $C_8$-hydrocarbon radical or an $-OR^7-$ radical,
$R^7$ represents a divalent linear or branched $C_2$- to $C_8$-hydrocarbon radical,
Q represents a group of general formula (VII)

$$-R^2-[NR^9(CH_2)_n]_iN(R^9)_2 \qquad (VII),$$

wherein i and n are as defined above,
$R^9$ may be identical or different and represents a hydrogen atom, an optionally fluorine-, chlorine-, bromine-, hydroxy- or $C_1$- to $C_5$-alkoxy-substituted $C_1$- to $C_{18}$-hydrocarbon radical or an acyl radical,
Y represents a radical of general formula (VIII) and/or (IX)

$$-OR^1 \text{ (VIII) and/or } -[O(CHR^{10})_p]_mOR^{11} \qquad (IX),$$

wherein
m is 0 or an integer from 1 to 100, preferably 1 to 20, and
p is 2, 3 or 4,
$R^1$ represents a hydrogen atom or a $C_1$- to $C_4$-alkyl radical,
$R^{10}$ represents a hydrogen atom or a $C_1$- to $C_{18}$-hydrocarbon radical,
$R^{11}$ represents a hydrogen atom, a $C_1$- to $C_{10}$-hydrocarbon radical, preferably a $C_4$- to $C_{10}$-hydrocarbon radical, or a group of formula $-(C{=}O)-R^{12}$,
$R^{12}$ represents a $C_1$- to $C_{10}$-hydrocarbon radical or an $-OR^{13}$ radical, and
$R^{13}$ represents a $C_1$- to $C_{10}$-hydrocarbon radical,
with the proviso that at least a portion of the radicals Y are radicals of formula (IX),
with the proviso that impurities of octamethylcyclotetrasiloxane (D4 cyclics), decamethylcyclopentasiloxane (D5

cyclics) and dodecamethylcyclohexasiloxane (D6 cyclics) are present in proportions of less than 0.1% by weight in each case and after a storage time of 20 days at a temperature of 50°C the proportions of D4 cyclics, representative of D4, D5 and D6, remain below 0.1% by weight in each case, based in each case on the total weight of the derivatives of amino-functional organopolysiloxanes.

2. Use according to Claim 1, **characterized in that** the compositions according to the invention employ derivatives of amino-functional organopolysiloxanes of general formula (X)

$$YR_2SiO\text{-}(R_2SiO)_k\text{-}(RZSiO)_l\text{-}(RQSiO)_o\text{-}R_2SiY \qquad (X),$$

wherein

R, Y, Z and Q are as defined in Claim 1,
k is an integer from 50 to 700,
l is an integer from 1 to 30 and
o is an integer from 0 to 30, preferably 1 to 15,
with the proviso that at least a portion of the radicals Y are radicals of formula (IX).

3. Use according to Claim 1 or 2, **characterized in that** a portion of the radicals Y corresponds to (iso)oxyalkyl radicals of formula (IX), wherein $C_4$-$C_{10}$-alkoxy radicals or $C_4$-$C_{10}$-monoalkyl glycol ether radicals are concerned, in addition to radicals of formula (VIII) which are hydroxyl and/or methoxy radicals.

4. Use according to Claims 1 to 3, **characterized in that** the compositions employed are aqueous emulsions containing low-cyclics derivatives of amino-functional organopolysiloxanes (A) according to Claims 1, 2 or 3, emulsifiers (B) and/or co-emulsifiers (B') and
water (C).

5. Process for washfast treatment of fibrous substrates according to any of Claims 1 to 4, **characterized in that** the compositions are applied to the fibrous substrates and the thus-treated fibrous substrates are allowed to dry at temperatures of preferably 20°C to 200°C.

6. Process according to Claim 5, **characterized in that** the fibrous substrates are textiles.

7. Process for producing the low-cyclics derivatives of amino-functional organopolysiloxanes employed in the compositions according to Claims 1 to 4, by reacting amino-functional organopolysiloxanes (1) containing amino-functional siloxane units of general formula (II)

$$R_cQ_dSiO_{\frac{4-(c+d)}{2}} \qquad (II),$$

wherein

Q, R, c and d are as defined in Claim 1,
and siloxane units of general formula (XII)

$$R_g(OR^1)_fSiO_{\frac{4-(f+g)}{2}} \qquad (XII),$$

wherein
R, $R^1$, f and g are as defined in Claim 1,
with compounds (2) of general formula (XI)

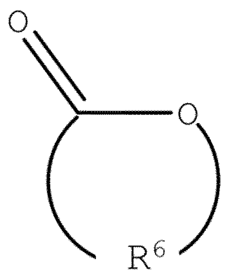

(XI),

wherein
$R^6$ is as defined in Claim 1,
and compounds (3) of general formula (XIII)

$$H\text{-}[O(CHR^{10})_p]_mOR^{11} \quad (XIII),$$

wherein
$R^{10}$, $R^{11}$, m and p are as defined in Claim 1,
with the proviso that in the obtained derivatives of amino-functional organopolysiloxanes the content of D4, D5 and D6 cyclics is less than 0.1% by weight in each case as a result of effecting distillative removal of the cyclics before, during or after the reaction.

8. Process according to Claim 7, **characterized in that** amino-functional organopolysiloxanes (1) containing amino-functional siloxane units of general formula (II) and siloxane units of general formula (XII) are simultaneously reacted with compounds (2) of general formula (XI) and compounds (3) of general formula (XIII).

9. Process according to Claim 7, **characterized in that** amino-functional organopolysiloxanes (1) containing amino-functional siloxane units of general formula (II) and siloxane units of general formula (XII) are reacted with compounds (2) of general formula (XI) in a first reaction step and the reaction products formed therefrom are subsequently reacted with compounds (3) of general formula (XIII) in a further reaction step.

10. Process according to Claim 7, **characterized in that** amino-functional organopolysiloxanes (1) containing amino-functional siloxane units of general formula (II) and siloxane units of general formula (XII) are reacted with compounds (3) of general formula (XIII) in a first reaction step and the reaction products formed therefrom are subsequently reacted with compounds (2) of general formula (XI) in a further reaction step.

11. Process for producing the low-cyclics derivatives of amino-functional organopolysiloxanes employed in the compositions according to Claims 1 to 4, by reacting amino-functional organopolysiloxanes (1) containing amino-functional siloxane units of general formula (II)

$$R_cQ_dSiO_{\frac{4-(c+d)}{2}} \quad (II),$$

wherein

Q, R, c and d are as defined in Claim 1,
and siloxane units of general formula (XII)

$$R_g(OR^1)_fSiO_{\frac{4-(f+g)}{2}} \quad (XII),$$

wherein
R, $R^1$, f and g are as defined in Claim 1,
with compounds (2) of general formula (XI) in a first step

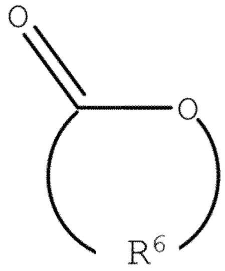

(XI),

wherein
$R^6$ is as defined in Claim 1,
and
subsequently effecting distillative removal of D4, D5 and D6 cyclics from the reaction product,
and reacting the purified reaction product from the first step with compounds (3) of general formula (XIII) in a second step

$$H\text{-}[O(CHR^{10})_p]_mOR^{11} \qquad (XIII),$$

wherein
$R^{10}$, $R^{11}$, m and p are as defined in Claim 1,
with the proviso that in the obtained derivatives of amino-functional organopolysiloxanes the content of D4, D5 and D6 cyclics is less than 0.1% by weight in each case.

**12.** Process according to Claims 7 to 11, **characterized in that** the amino-functional organopolysiloxanes (1) employed are those of general formula (XIV),

$$R^1OR_2SiO\text{-}(R_2SiO)_k\text{-}(RQSiO)_{l+o}\text{-}SiR_2OR^1 \qquad (XIV),$$

wherein
R and $R^1$ are as defined in Claim 1 and k, l and o are as defined in Claim 2.

**13.** Process according to Claims 7 to 12, **characterized in that** the compounds (2) of general formula (XI) employed are lactones or cyclic carbonic esters.

**14.** Process according to Claims 7 to 13, **characterized in that** the compounds (3) of general formula (XIII) employed are a $C_4$- to $C_{10}$-alcohol or a $C_4$- to $C_{10}$-monoalkyl glycol ether.

**15.** Process according to Claims 7 to 14, **characterized in that** the compounds (2) of general formula (XI) employed are $\gamma$-butyrolactone or propylene carbonate.

**Revendications**

**1.** Utilisation de compositions pour l’apprêtage hydrophile et résistant au lavage donnant un toucher doux de substrats fibreux contenant des dérivés d’organopolysiloxanes à fonction amine, contenant des unités siloxane de formule générale (I)

$$R_aZ_bSiO_{\frac{4-(a+b)}{2}} \qquad (I),$$

dans laquelle

a est 0, 1 ou 2, de préférence 0 ou 1,
b est 1, 2 ou 3, de préférence 1,
étant entendu que la somme de a+b est $\leq 3$,
éventuellement des unités siloxane de formule générale (II)

$$R_cQ_dSiO_{\frac{4-(c+d)}{2}} \qquad (II),$$

dans laquelle
c est 0, 1 ou 2, de préférence 0 ou 1,
d est 1, 2 ou 3, de préférence 1,
étant entendu que la somme de c+d est ≤ 3,
des unités siloxane de formule générale (III)

$$R_eSiO_{\frac{4-e}{2}} \qquad (III),$$

dans laquelle
e est 0, 1, 2 ou 3, de préférence 2,
et des unités siloxane de formule générale (IV)

$$Y_fR_gSiO_{\frac{4-(f+g)}{2}} \qquad (IV),$$

dans laquelle
f est 1, 2 ou 3, de préférence 1,
g est 0, 1 ou 2, de préférence 2,
étant entendu que la somme de f+g est ≤ 3,
R peut être le même ou différent, et représente un atome d'hydrogène ou un radical hydrocarboné monovalent en $C_1$-$C_{18}$ éventuellement substitué par le fluor, le chlore ou le brome,
Z représente un groupe de formule générale (V)

$$-R^2-[NR^3(CH_2)_n]_iNR^4R^5 \qquad (V),$$

dans laquelle
i est 0, 1, 2, 3 ou 4,
n est 2, 3, 4, 5 ou 6,
$R^2$ représente un radical hydrocarboné divalent en $C_1$-$C_{18}$ linéaire ou ramifié,
$R^3$ représente un atome d'hydrogène, un radical hydrocarboné en $C_1$-$C_{18}$ éventuellement substitué par le fluor, le chlore, le brome ou par hydroxy ou alcoxy en $C_1$-$C_5$, ou un radical acyle ou un radical de formule générale (VI),
$R^4$ représente un radical de formule générale (VI)

$$-(C{=}O)-R^6-OH \qquad (VI),$$

$R^5$ représente un atome d'hydrogène ou un radical hydrocarboné en $C_1$-$C_{18}$ éventuellement substitué par le fluor, le chlore, le brome ou par hydroxy ou alcoxy en $C_1$-$C_5$ ou un radical acyle,
$R^6$ représente un radical hydrocarboné divalent en $C_2$-$C_8$ linéaire ou ramifié ou un radical $-OR^7-$,
$R^7$ représente un radical hydrocarboné divalent en $C_2$-$C_8$ linéaire ou ramifié,
Q représente un groupe de formule générale (VII)

$$-R^2-[NR^9(CH_2)_n]_iN(R^9)_2 \qquad (VII),$$

dans laquelle i et n ont les significations indiquées plus haut pour ces indices,
$R^9$ peut être le même ou différent, et représente un atome d'hydrogène, un radical hydrocarboné en $C_1$-$C_{18}$ éventuellement substitué par le fluor, le chlore, le brome ou par hydroxy ou alcoxy en $C_1$-$C_5$ ou un radical acyle,
Y représente un radical de formule générale (VIII) et/ou de formule générale (IX)

$$-OR^1 \text{ (VIII) et/ou } -[O(CHR_{10})_p]_mOR^{11} \qquad (IX)$$

formules dans lesquelles
m est 0 ou un nombre entier valant de 1 à 100, de préférence 1 à 20,
p est 2, 3 ou 4,
$R^1$ représente un atome d'hydrogène ou un radical alkyle en $C_1$-$C_4$,
$R^{10}$ représente un atome d'hydrogène ou un radical hydrocarboné en $C_1$-$C_{18}$,
$R^{11}$ représente un atome d'hydrogène, un radical hydrocarboné en $C_1$-$C_{10}$, de préférence un radical hydrocarboné en $C_4$-$C_{10}$, ou un groupe de formule -(C=O)-$R^{12}$,
$R^{12}$ représente un radical hydrocarboné en $C_1$-$C_{10}$ ou un radical -O-$R^{13}$, et
$R^{13}$ représente un radical hydrocarboné en $C_1$-$C_{10}$,
étant entendu qu'au moins une partie des radicaux Y sont de formule (IX),
étant entendu que des impuretés consistant en octaméthylcyclotétrasiloxane (cyclène D4), décaméthyl-cyclopentasiloxane (cyclène D5) et dodécaméthylcyclo-hexasiloxane (cyclène D6) sont contenues en des proportions de chaque fois moins de 0,1 % en poids et après un temps de stockage de 20 jours à une température de 50 °C les proportions de cyclène D4, représentatif de D4, D5 et D6, sont toujours inférieures à chaque fois 0,1 % en poids, chaque fois par rapport au poids total des dérivés d'organopolysiloxanes à fonction amine.

2. Utilisation selon la revendication 1, **caractérisée en ce que** dans les compositions selon l'invention sont utilisés des dérivés d'organopolysiloxanes à fonction amine de formule générale (X)

$$YR_2SiO\text{-}(R_2SiO)_k\text{-}(RZSiO)_l\text{-}(RQSiO)_o\text{-}R_2SiY \qquad (X)$$

dans laquelle

R, Y, Z et Q ont les significations indiquées pour ces symboles dans la revendication 1,
k est un nombre entier valant de 50 à 700,
l est un nombre entier valant de 1 à 30 et
o est un nombre entier valant de 0 à 30, de préférence 1 à 15,
étant entendu qu'au moins une partie des radicaux Y sont de formule (IX).

3. Utilisation selon la revendication 1 ou 2, **caractérisée en ce qu'**une partie des radicaux Y correspond à des radicaux (iso)oxyalkyle de formule (IX), pour ce qui est desquels il s'agit de radicaux alcoxy en $C_4$-$C_{10}$ ou de radicaux monoalkyl-($C_4$-$C_{10}$)glycoléther, en plus de radicaux de formule (VIII), qui sont des radicaux hydroxy et/ou méthoxy.

4. Utilisation selon la revendication 1 à la revendication 3, **caractérisée en ce qu'**en tant que compositions sont utilisées des émulsions aqueuses contenant des dérivés pauvres en cyclènes d'organopolysiloxanes à fonction amine (A) selon la revendication 1, 2 ou 3, des émulsifiants (B) et/ou des co-émulsifiants (B') et de l'eau (C).

5. Procédé pour l'apprêtage permanent au lavage de substrats fibreux selon l'une quelconque des revendications 1 à 4, **caractérisé en ce que** les compositions sont appliquées sur les substrats fibreux et les substrats fibreux ainsi traités sont laissés sécher à des températures de préférence de 20 à 200 °C.

6. Procédé selon la revendication 5, **caractérisé en ce que** les substrats fibreux sont des textiles.

7. Procédé pour la préparation des dérivés pauvres en cyclènes d'organopolysiloxanes à fonction amine selon la revendication 1 à la revendication 4, utilisés dans les compositions, par mise en réaction d'organopolysiloxanes à fonction amine (1), contenant des unités siloxane à fonction amine de formule générale (II),

$$R_cQ_dSiO_{\frac{4-(c+d)}{2}} \qquad (II),$$

dans laquelle

Q, R, c et d ont les significations indiquées pour ces symboles dans la revendication 1 ;
et des unités siloxane de formule générale (XII)

$$R_g(OR^1)_fSiO_{\frac{4-(f+g)}{2}} \qquad (XII),$$

dans laquelle
R, $R^1$, f et g ont les significations indiquées pour ces symboles dans la revendication 1,
avec des composés (2) de formule générale (XI)

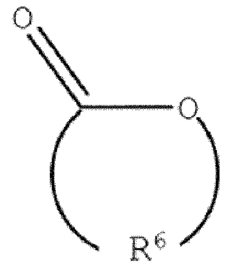

(XI),

dans laquelle
$R^6$ a la signification indiquée pour ce symbole dans la revendication 1,
et des composés (3) de formule générale (XIII)

$$H\text{-}[O(CHR^{10})_p]_mOR^{11} \qquad (XIII),$$

dans laquelle
$R^{10}$, $R^{11}$, m et p ont les significations indiquées pour ces symboles dans la revendication 1,
étant entendu que dans les dérivés obtenus d'organopolysiloxanes à fonction amine la teneur en cyclènes D4, D5 et D6 est chaque fois inférieure à 0,1 % en poids, par le fait que les cyclènes sont éliminés par distillation avant, pendant ou après la réaction.

**8.** Procédé selon la revendication 7, **caractérisé en ce que** sont mis en réaction simultanément des organopolysiloxanes à fonction amine (1) contenant des unités siloxane à fonction amine de formule générale (II) et des unités siloxane de formule générale (XII) avec des composés (2) de formule générale (XI) et des composés (3) de formule générale (XIII).

**9.** Procédé selon la revendication 7, **caractérisé en ce que** des organopolysiloxanes à fonction amine (1), contenant des unités siloxane à fonction amine de formule générale (II) et des unités siloxane de formule générale (XII) sont mis à réagir dans une première étape de réaction avec des composés (2) de formule générale (XI), et les produits de réaction formés à partir de ceux-ci sont ensuite mis à réagir dans une étape de réaction ultérieure avec des composés (3) de formule générale (XIII).

**10.** Procédé selon la revendication 7, **caractérisé en ce que** des organopolysiloxanes à fonction amine (1), contenant des unités siloxane à fonction amine de formule générale (II) et des unités siloxane de formule générale (XII) sont mis à réagir dans une première étape de réaction avec des composés (3) de formule générale (XIII), et les produits de réaction formés à partir de ceux-ci sont ensuite mis à réagir dans une étape de réaction ultérieure avec des composés (2) de formule générale (XI).

**11.** Procédé pour la préparation des dérivés pauvres en cyclènes d'organopolysiloxanes à fonction amine selon la revendication 1 à la revendication 4, utilisés dans les compositions, par mise en réaction d'organopolysiloxanes à fonction amine (1), contenant des unités siloxane à fonction amine de formule générale (II),

$$R_cQ_dSiO_{\frac{4-(c+d)}{2}} \qquad (II),$$

dans laquelle

Q, R, c et d ont les significations indiquées pour ces symboles dans la revendication 1 ; et des unités siloxane de formule générale (XII)

$$R_g(OR^1)_f SiO_{\frac{4-(f+g)}{2}} \quad (XII),$$

dans laquelle
R, $R^1$, f et g ont les significations indiquées pour ces symboles dans la revendication 1, dans une première étape avec des composés (2) de formule générale (XI)

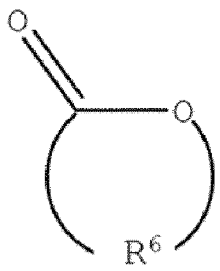

(XI),

dans laquelle
$R^6$ a la signification indiquée pour ce symbole dans la revendication 1 et ensuite les cyclènes D4 D5 et D6 sont éliminés du produit de réaction par distillation, et dans une 2<sup>e</sup> étape le produit de réaction purifié provenant de la première étape est mis à réagir avec des composés (3) de formule générale (XIII)

$$H\text{-}[O(CHR^{10})_p]_m OR^{11} \quad (XIII),$$

dans laquelle
$R^{10}$, $R^{11}$, m et p ont les significations indiquées pour ces symboles dans la revendication 1, étant entendu que dans les dérivés obtenus d'organopolysiloxanes à fonction amine la teneur en cyclènes D4, D5 et D6 est chaque fois inférieure à 0,1 % en poids.

**12.** Procédé selon la revendication 7 à la revendication 11, **caractérisé en ce qu'**en tant qu'en tant qu'organopolysiloxanes à fonction amine (1) sont utilisés ceux de formule générale (XIV),

$$R^1OR_2SiO\text{-}(R_2SiO)_k\text{-}(RQSiO)_{l+o}\text{-}SiR_2OR^1 \quad (XIV),$$

dans laquelle
R et $R^1$ ont les significations indiquées pour ces symboles dans la revendication 1 et k, l et o ont les significations indiquées pour ces indices dans la revendication 2.

**13.** Procédé selon la revendication 7 à la revendication 12, **caractérisé en ce qu'**en tant que composés (2) de formule générale (XI) sont utilisé(e)s des lactones ou des esters cycliques d'acides carboxyliques.

**14.** Procédé selon la revendication 7 à la revendication 13, **caractérisé en ce qu'**en tant que composés (3) de formule générale (XIII) est utilisé un alcool en $C_4$-$C_{10}$ ou un monoalkyl ($C_4$-$C_{10}$) glycoléther.

**15.** Procédé selon la revendication 7 à la revendication 14, **caractérisé en ce qu'**en tant que composés (2) de formule générale (XI) on utilise la $\gamma$-butyrolactone ou le carbonate de propylène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5981681 A
- US 7041767 B2
- US 7897716 B2
- US 20080075683 A1
- US 6576606 B2
- US 4978561 A
- US 5174813 A
- US 5389364 A
- US 5824814 A
- KR 20070072069 A
- EP 0856553 A1
- US 7129369 B2
- US 3775452 A


### In der Beschreibung aufgeführte Nicht-Patentliteratur


- **G. ENGELHARDT ; H. JANCKE.** Über die H-, C- und Si-NMR chemischen Verschiebungen einiger linearer, verzweigter und cyclischer Methyl-Siloxan-Verbindungen. *J. Organometal. Chem,* 1971, vol. 28, 293-300
- Chapter 8 NMR spectroscopy of organosilicon compounds. **ELIZABETH A. WILLIAMS.** The Chemistry of Organic Silicon Compounds. John Wiley and Sons Ltd, 1989, 511-533